(19) Europäisches Patentamt
European Patent Office
Office européen des brevets

(11) **EP 4 292 694 A1**

(12) **EUROPÄISCHE PATENTANMELDUNG**

(43) Veröffentlichungstag:
20.12.2023  Patentblatt 2023/51

(21) Anmeldenummer: 23176800.3

(22) Anmeldetag: 01.06.2023

(51) Internationale Patentklassifikation (IPC):
**B01D 63/16** (2006.01)   **B01D 65/08** (2006.01)
**B01D 33/00** (2006.01)   **B01D 46/00** (2022.01)
**B01D 46/26** (2006.01)

(52) Gemeinsame Patentklassifikation (CPC):
**B01D 63/16; B01D 33/067; B01D 33/073;
B01D 33/801; B01D 46/0056; B01D 46/26;
B01D 65/08;** B01D 2311/2607; B01D 2311/2688;
B01D 2315/02

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(30) Priorität: **16.06.2022  EP 22179441**

(71) Anmelder: **Levitronix GmbH
8048 Zürich (CH)**

(72) Erfinder: **Schöb, Reto
8832 Wollerau (CH)**

(74) Vertreter: **IPS Irsch AG
Langfeldstrasse 88
8500 Frauenfeld (CH)**

(54) **ROTATIONSFILTERSYSTEM, ROTATIONSFILTERVORRICHTUNG SOWIE SEPARATIONSSYSTEM**

(57)    Es wird ein Rotationsfiltersystem zum Ausfiltern eines Filtrats aus einem Fluid vorgeschlagen, mit einer Rotationsfiltervorrichtung (10) und einer Antriebsvorrichtung (100), wobei die Rotationsfiltervorrichtung (10) ein stationäres Filtergehäuse (2) und eine um eine axiale Richtung (A) rotierbare Filtereinheit (3) umfasst, wobei das Filtergehäuse (2) einen Einlass (23) für das Fluid (F), einen ersten Auslass (21) zum Abführen des Filtrats (P), sowie einen zweiten Auslass (22) zum Abführen eines Retentats (R) aufweist, wobei die Filtereinheit (3) in dem Filtergehäuse (2) angeordnet und vollständig von dem Filtergehäuse (2) umschlossen ist, wobei die Filtereinheit (3) ein Filterelement (4) aufweist, welches einen Fluidraum (41) von einem Filtratraum (42) abgrenzt, wobei das Filtrat (P) aus dem Filtratraum (42) durch den ersten Auslass (21) abführbar ist, wobei die Filtereinheit (3) ferner einen magnetisch wirksamen Kern (6, 6') umfasst, welcher scheiben- oder ringförmig ausgestaltet ist, und wobei die Antriebsvorrichtung (100) ein Antriebsgehäuse (101) aufweist, in welchem ein Stator (102, 102') zum Antreiben der Rotation der Filtereinheit (3) vorgesehen ist. Der Stator (102, 102') ist als Lager- und Antriebsstator ausgestaltet ist, der mit dem magnetisch wirksamen Kern (6, 6') der Filtereinheit (3) als elektromagnetischer Drehantrieb zusammenwirkt, sodass die Filtereinheit (3) berührungslos magnetisch antreibbar und bezüglich des Stators (102, 102') magnetisch lagerbar ist. Ferner wird eine Rotationsfiltervorrichtung und ein Separationssystem vorgeschlagen.

Fig.1

EP 4 292 694 A1

**Beschreibung**

Rotationsfiltersystem, Rotationsfiltervorrichtung sowie Separationssystem

Die Erfindung betrifft ein Rotationsfiltersystem zum Ausfiltern eines Filtrats aus einem Fluid sowie ein Separationssystem für einen Bioreaktor zur Extraktion einer Substanz aus einem in dem Bioreaktor vorrätigen Fluid gemäss dem Oberbegriff des unabhängigen Patentanspruchs der jeweiligen Kategorie. Die Erfindung betrifft ferner eine Rotationsfiltervorrichtung für ein solches Rotationsfiltersystem.

In der biotechnologischen und in der pharmazeutischen Industrie werden häufig Bioreaktoren zur Gewinnung von Substanzen, beispielsweise Proteinen, oder zur Züchtung von Zellen oder anderem biologischen Material eingesetzt. Dabei können Bioreaktoren sowohl in kontinuierlichen Prozessen als auch in Batch Prozessen betrieben werden. Der Betrieb in kontinuierlichen Prozessen wird üblicherweise als Perfusionsbetrieb bezeichnet und der dabei verwendeten Bioreaktor als Perfusions-Bioreaktor. So sind beispielsweise Perfusionsverfahren mit Bioreaktoren bekannt, die zur kontinuierlichen Kultivierung von Zellen eingesetzt werden, wobei beispielsweise Stoffwechselprodukte der Zellen mittels Filtration separiert werden und die Zellen in den Bioreaktor zurückgeführt werden. Dabei kann dem Bioreaktor beispielsweise kontinuierlich eine Nährlösung für die Zellen zugeführt werden, wodurch die Masse oder das Volumen der ausgefilterten Komponenten ersetzt werden.

Bei diesen Verfahren ist es eine übliche Methode, das Fluid, beispielsweise eine Zellbrühe (cell broth), dem Bioreaktor zu entnehmen, einer Filtervorrichtung zuzuführen, und das Retentat wieder in den Bioreaktor zurückzuführen. Die zu extrahierende Substanz wird dann als Filtrat bzw. als Permeat der Filtervorrichtung entnommen und abgeführt. Es sind zahlreiche Verfahren für solche Filterprozesse bekannt. Beispielsweise sind Rotationsfiltervorrichtungen bekannt, bei welchen die Filtermembran, welche den Filtratraum begrenzt, aus dem das Filtrat abgeführt wird, um eine axiale Richtung rotiert, beispielsweise mit einer Geschwindigkeit im Bereich von 100 Umdrehungen pro Minute. Der grosse Vorteil dieser Rotationsfiltervorrichtungen ist es, dass die Filtermembran viel weniger anfällig für Verstopfungen (clotting) beispielsweise durch im Fluid enthaltene Feststoffe ist. Durch die Rotation der Filtermembran werden Zentrifugalkräfte generiert, welche zum Wegschleudern von Ablagerungen in der oder auf der Filtermembran führen.

Da in Bioreaktoren biologische Aktivitäten stattfinden, ist für viel Prozesse die Sterilität von sehr grosser Bedeutung. Das Sterilisieren der Vorrichtungen, beispielsweise mittels Dampfsterilisation, stellt sehr häufig einen zeit- und kostenintensiven Faktor dar. Deshalb besteht heute die zunehmende Tendenz, für solche Prozesse mit Bioreaktoren und speziell auch mit Perfusions-Bioreaktoren, Komponenten der Vorrichtung als Einmalteile auszugestalten, um aufwändige Sterilisationsprozesse zu vermeiden oder auf ein Minimum zu reduzieren. Insbesondere werden diejenigen Komponenten, die während des Prozesses mit den biologischen Substanzen in direkten Kontakt kommen, häufig als Einmalteile ausgestaltet. Der Begriff Einmalteile (single use) bezeichnet dabei Teile bzw. Komponenten, die bestimmungsgemäss nur einmal benutzt werden dürfen. Nach der Anwendung werden die Einmalteile entsorgt und für die nächste Anwendung durch neue, das heisst noch nicht gebrauchte Einmalteile ersetzt.

Es gibt aber auch Probleme hinsichtlich der Sterilität, die nicht nur durch die Verwendung von Einmalkomponenten gelöst werden können. So sind beispielsweise bei Rotationsfiltervorrichtungen dynamische Dichtungen, beispielsweise Wellendichtungen, vorgesehen, mit denen im Betriebszustand die Welle, welche die Rotation des Filterelements antreibt, gegenüber dem statischen Filtergehäuse abgedichtet wird. Solche dynamischen Dichtungen können einerseits zu Leckagen führen, sowohl in dem Sinne, dass Substanzen ungewollt aus dem Prozess in die Umgebung austreten, als auch in dem Sinne, dass Verunreinigungen durch diese Dichtungen in den Prozess eindringen können. Andererseits können Verschleisserscheinungen oder Abrieb in den dynamischen Dichtungen dazu führen, dass unerwünschte Fremdstoffe in den Prozess eindringen und zu Verunreinigungen in den biologischen Prozessen führen, welche sogar die Brauchbarkeit des angestrebten Endproduktes gefährden können.

Ausgehend von diesem Stand der Technik ist es daher eine Aufgabe der Erfindung, ein Rotationsfiltersystem zum Ausfiltern eines Filtrats aus einem Fluid vorzuschlagen, das eine höhere Betriebssicherheit im Hinblick auf Leckagen und damit auch im Hinblick auf die Sterilität ermöglicht. Ferner ist es eine Aufgabe der Erfindung, eine Rotationsfiltervorrichtung für ein solches Rotationsfiltersystem vorzuschlagen. Eine weitere Aufgabe der Erfindung ist es, ein Separationssystem für einen Bioreaktor vorzuschlagen, bei welchem ein solches Rotationsfiltersystem zur Extraktion einer Substanz aus einem Fluid dient, welches in dem Bioreaktor vorrätig ist.

Die diese Aufgabe lösenden Gegenstände der Erfindung sind durch die Merkmale des unabhängigen Patentanspruchs der jeweiligen Kategorie gekennzeichnet.

Erfindungsgemäss wird also ein Rotationsfiltersystem zum Ausfiltern eines Filtrats aus einem Fluid vorgeschlagen, mit einer Rotationsfiltervorrichtung und einer Antriebsvorrichtung, wobei die Rotationsfiltervorrichtung ein stationäres Filtergehäuse und eine um eine axiale Richtung rotierbare Filtereinheit umfasst, wobei das Filtergehäuse einen Einlass für das Fluid, einen ersten Auslass zum Abführen des Filtrats, sowie einen zweiten Auslass zum Abführen eines Retentats aufweist, wobei die Filtereinheit in dem Filtergehäuse angeordnet und vollständig von dem Filtergehäuse umschlossen ist, wobei die Filtereinheit ein Filterelement aufweist, welches einen Fluidraum von einem Filtratraum abgrenzt, wobei das Filtrat aus dem Filtratraum durch den ersten Auslass abführbar ist, wobei die Filtereinheit ferner einen magnetisch wirksamen Kern umfasst, welcher scheiben- oder ringförmig ausgestaltet ist, und wobei die Antriebsvorrichtung ein Antriebsgehäuse aufweist, in welchem ein Stator zum Antreiben der Rotation der Filtereinheit vorgesehen ist. Der Stator ist als Lager- und Antriebsstator ausgestaltet, der mit dem magnetisch wirksamen Kern der Filtereinheit als elektromagnetischer Drehantrieb zusammenwirkt, sodass die Filtereinheit berührungslos magnetisch antreibbar und bezüglich des Stators magnetisch lagerbar ist.

Dadurch, dass die Filtereinheit den magnetisch wirksamen Kern umfasst, welcher mit dem Antriebsstator als elektromagnetischer Drehantrieb zusammenwirkt, mit welchem die Filtereinheit zudem magnetisch bezüglich des Stator lagerbar ist, bedarf es keiner rotierenden Welle mehr, welche aus dem Filtergehäuse herausgeführt werden müsste. Folglich bedarf es am stationären Filtergehäuse auch keiner dynamischen Dichtung mehr, welche das Filtergehäuse am Durchtritt einer rotierenden Welle abdichten müsste. Durch den berührungslosen magnetischen Antrieb der Rotation der Filtereinheit und deren magnetische Lagerung bezüglich des Stators kann das Filtergehäuse also erheblich dichter und insbesondere ohne Wellendichtung zum Abdichten des Innenraums des Filtergehäuses gegenüber der Umgebung ausgestaltet werden. Somit besteht auch keine Gefahr, dass ungewollt Fluid über eine Leckage aus dem Filtergehäuse austritt, oder dass über eine Leckage an einer Dichtung ungewollt Verunreinigungen oder andere Substanzen in das Filtergehäuse eintreten. Durch den Verzicht auf eine dynamischen Wellendichtung am Filtergehäuse wird ferner die Gefahr vermieden, dass Verschleissprodukte, beispielsweise Abrieb, in das Filtergehäuse eindringen und zu Verunreinigungen führen.

Vorzugsweise ist das Filtergehäuse zum hermetischen Umschliessen der Filtereinheit ausgestaltet, sodass Substanzen, wie beispielsweise das Fluid, das Retentat und das Filtrat ausschliesslich durch den Einlass und die beiden Auslässe in das Filtergehäuse eingebracht werden können bzw. aus dem Filtergehäuse abgeführt werden können. Ansonsten ist das Filtergehäuse hermetisch dicht.

Gemäss einer bevorzugten Ausgestaltung ist zwischen der rotierbaren Filtereinheit und dem ersten Auslass des Filtergehäuses eine berührungslose Dichtung vorgesehen ist, wobei die berührungslose Dichtung im Innenraum des Filtergehäuses angeordnet ist. Die berührungslose Dichtung im Inneren des Filtergehäuses dichtet zwischen dem im Betriebszustand rotierenden Filterelement und dem stationären ersten Auslass, durch welchen das Filtrat aus dem Filtratraum abgeführt wird. Die berührungslose Dichtung, die beispielsweise als Labyrinthdichtung ausgestaltet ist, reduziert oder minimiert denjenigen Leckagestrom des Fluids, der vom Einlass direkt - also ohne Passieren des Filterelements - in den Filtratraum fliesst.

Eine weitere bevorzugte Massnahme besteht darin, dass auf der Filtereinheit und benachbart zu der berührungslosen Dichtung Pumpenflügel zum Fördern des Fluids vorgesehen sind, wobei die Pumpenflügel zur Rotation um die axiale Richtung ausgestaltet und drehfest mit der Filtereinheit verbunden sind. Die Pumpenflügel dienen dazu, in der Nachbarschaft der berührungslosen Dichtung einen Druck aufzubauen und damit den Leckagestrom durch die berührungslose Dichtung zu reduzieren. Je nach Ausgestaltung können die Pumpenflügel zusätzlich auch dazu dienen, die Zirkulation des Fluid bzw. des Retentats anzutreiben. Ist das Rotationsfiltersystem beispielsweise mit einem Bioreaktor verbunden, so können die Pumpenflügel zumindest dazu beitragen, das Fluid aus dem Bioreaktor durch den Einlass anzusaugen und das Retentat durch den zweiten Auslass zum Bioreaktor zu rezirkulieren. Je nach Ausgestaltung können die Pumpenflügel auch die einzige Pumpvorrichtung zum Zirkulieren des Fluids bzw. des Retentats sein.

Gemäss einer bevorzugten Ausführungsform sind an einer Aussenseite der Filtereinheit Schaufeln zum Erzeugen eines Transmembrandrucks über das Filterelement vorgesehen, wobei die Schaufeln zur Rotation um die axiale Richtung ausgestaltet und drehfest mit der Filtereinheit verbunden sind. Diese Schaufeln dienen dazu, den Transmembrandruck, also die Druckdifferenz zwischen dem Fluidraum und dem Filtratraum über das Filterelement, einzustellen bzw. zu vergrössern. Die Schaufel können beispielsweise an der radial aussenliegenden Umfangsfläche der Filtereinheit und/oder an einer Stirnfläche der Filtereinheit angeordnet sein.

In einer bevorzugten Ausgestaltung weist das Filtergehäuse eine Stirnfläche auf, an welcher der Einlass, der erste Auslass und der zweite Auslass angeordnet sind. Somit sind alle Fluidöffnungen des Filtergehäuses, nämlich der Einlass und die beiden Auslässe, an der gleichen Stirnfläche des Filtergehäuses vorgesehen. Diese Ausgestaltung ist insbesondere vorteilhaft, wenn das Antriebsgehäuse eine zentral angeordnete Kavität, beispielsweise eine topf- oder becherförmige Kavität, aufweist, in welche das Filtergehäuse eingesetzt wird.

Gemäss einen bevorzugten Ausführungsbeispiel umfasst die Filtereinheit zwei magnetisch wirksame Kerne, von denen jeder scheiben- oder ringförmig ausgestaltet ist, und die bezüglich der axialen Richtung beabstandet zueinander angeordnet sind, wobei im Antriebsgehäuse der Antriebsvorrichtung zwei Statoren zum Antreiben der Rotation der Filtereinheit vorgesehen sind, wobei jeder Stator als Lager- und Antriebsstator ausgestaltet ist, der mit jeweils einem der magnetisch wirksamen Kerne der Filtereinheit als elektromagnetischer Drehantrieb zusammenwirkt, sodass die Filtereinheit berührungslos magnetisch antreibbar und bezüglich der Statoren magnetisch lagerbar ist. Somit sind zwei Antriebs- und Lagerstellen für den Antrieb der Rotation und für die magnetische Lagerung der Filtereinheit vorgesehen, welche bezüglich der axialen Richtung beabstandet sind. Hiermit lässt sich einerseits ein höheres Drehmoment für den Antrieb des Filterelements erzeugen, und andererseits kann die magnetische Lagerung und insbesondere die Stabilisierung gegen Verkippungen verbessert werden. Vorzugsweise sind dabei die beiden magnetischen Kerne koaxial und somit auch parallel zueinander angeordnet. Ferner ist es vorteilhaft, den Abstand der beiden magnetisch wirksamen Kerne bezüglich der axialen Richtung möglichst gross zu gestalten, also die magnetisch wirksamen Kerne an oder in der Nähe der beiden Stirnflächen anzuordnen, welche die Filtereinheit bezüglich der axialen Richtung begrenzen.

In einer bevorzugten Ausführungsform sind die Rotationsfiltervorrichtung und die Antriebsvorrichtung so ausgestaltet, dass das stationäre Filtergehäuse in das Antriebsgehäuse einsetzbar ist, und jeder elektromagnetische Drehantrieb als Innenläufer ausgestaltet ist. Bei Ausgestaltungen mit nur einem magnetisch wirksamen Kern in der Filtereinheit ist dann also der Stator im Antriebsgehäuse so angeordnet, dass er den magnetisch wirksamen Kern radial aussenliegend umgibt, wenn das Filtergehäuse in das Antriebsgehäuse eingesetzt ist. Bei Ausgestaltungen mit zwei magnetisch wirksamen Kernen in der Filtereinheit sind dann die Statoren im Antriebsgehäuse so angeordnet, dass jeder Stator jeweils einen der magnetisch wirksamen Kerne radial aussenliegend umgibt, wenn das Filtergehäuse in das Antriebsgehäuse eingesetzt ist.

In einer anderen ebenfalls bevorzugten Ausführungsform sind die Rotationsfiltervorrichtung und die Antriebsvorrichtung so ausgestaltet, dass das Antriebsgehäuse in eine zentrale Ausnehmung des stationären Filtergehäuses einsetzbar ist, und jeder elektromagnetische Drehantrieb als Aussenläufer ausgestaltet ist. Bei Ausgestaltungen mit nur einem magnetisch wirksamen Kern in der Filtereinheit ist dann also der Stator im Antriebsgehäuse so angeordnet, dass der magnetisch wirksame Kern den Stator radial aussenliegend umgibt, wenn das Antriebsgehäuse in das Filtergehäuse eingesetzt ist. Bei Ausgestaltungen mit zwei magnetisch wirksamen Kernen in der Filtereinheit sind dann die Statoren im Antriebsgehäuse so angeordnet, dass jeder magnetisch wirksame Kern jeweils einen der Statoren radial aussenliegend umgibt, wenn das Antriebsgehäuse in das Filtergehäuse eingesetzt ist.

Im Hinblick auf die Sterilität ist es besonders bevorzugt, dass die Rotationsfiltervorrichtung als Einmalvorrichtung für den Einmalgebrauch ausgestaltet ist. Die Rotationsfiltervorrichtung kann also bestimmungsgemäss nur genau einmal gebraucht werden und muss für die nächste Anwendung durch eine neue, ungebrauchte Rotationsfiltervorrichtung ersetzt werden. Die Antriebsvorrichtung ist vorzugsweise als wiederverwendbare Vorrichtung für den Mehrfachgebrauch ausgestaltet, wobei das Filtergehäuse in das Antriebsgehäuse einsetzbar ist, oder das Filtergehäuse eine zentrale Ausnehmung zur Aufnahme des Antriebsgehäuses aufweist. Da die Antriebsvorrichtung nur Komponenten enthält, welche nicht in direkten körperlichen Kontakt mit dem Fluid, mit dem Retentat oder mit dem Filtrat kommen, kann in der Regel auf eine Sterilität bzw. auf eine Sterilisierung der Antriebsvorrichtung verzichtet werden.

Durch die Erfindung wird ferner eine Rotationsfiltervorrichtung für ein Rotationsfiltersystem vorgeschlagen, welches gemäss der Erfindung ausgestaltet ist, wobei das Filtergehäuse in das Antriebsgehäuse einsetzbar ist, oder das Filtergehäuse eine zentrale Ausnehmung zur Aufnahme des Antriebsgehäuses aufweist. Die Rotationsfiltervorrichtung stellt dabei üblicherweise Verbrauchsmaterial dar, während die Antriebsvorrichtung die wiederverwendbaren Komponenten für den Antrieb und die magnetische Lagerung der Filtereinheit in dem Filtergehäuse umfasst.

Vorzugsweise ist die Rotationsfiltervorrichtung als Einmalvorrichtung für den Einmalgebrauch ausgestaltet.

Durch die Erfindung wird ferner ein Separationssystem für einen Bioreaktor zur Extraktion einer Substanz aus einem in dem Bioreaktor vorrätigen Fluid vorgeschlagen, wobei das Separationssystem eine Rotationsfiltersystem umfasst, welches einen Einlass für das Fluid, einen ersten Auslass zum Abführen eines Filtrats, sowie einen zweiten Auslass zum Abführen eines Retentats aufweist, wobei eine erste Strömungsverbindung vorgesehen ist, mit welcher der Einlass mit dem Bioreaktor verbindbar ist, wobei eine zweite Strömungsverbindung vorgesehen ist, mit welcher der zweite Auslass mit dem Bioreaktor verbindbar ist, wobei die Substanz als das Filtrat durch den ersten Auslass entnehmbar ist, und wobei eine Pumpvorrichtung zum Zirkulieren des Fluids und des Retentats durch die erste Strömungsverbindung und die zweite Strömungsverbindung vorgesehen ist. Das Rotationsfiltersystem ist dabei gemäss der Erfindung ausgestaltet.

Gemäss einer bevorzugten Ausgestaltung ist die Pumpvorrichtung in das Rotationsfiltersystem integriert. Dabei ist es möglich, dass die Pumpvorrichtung die einzige Vorrichtung zum Fördern bzw. zum Zirkulieren des Fluids und des Retentats ist. Es sind aber auch Ausgestaltungen möglich, bei denen zusätzlich in der ersten und/oder in der zweiten Strömungsverbindung eine oder mehrere Pumpe(n) vorgesehen sind, die nicht integraler Bestandteil des Rotationsfiltersystems sind.

Vorzugsweise ist die Pumpvorrichtung an der rotierbaren Filtereinheit vorgesehen.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen (teilweise im Schnitt):

Fig. 1:          eine schematische Darstellung eines
                 ersten Ausführungsbeispiels eines erfindungsgemässen Rotationsfiltersystems,

Fig. 2: eine schematische Darstellung eines zweiten Ausführungsbeispiels eines erfindungsgemässen Rotationsfiltersystems,

Fig. 3: einen Schnitt durch einen der Statoren des zweiten Ausführungsbeispiels in einem Schnitt senkrecht zur axialen Richtung entlang der Schnittlinie III-III in Fig. 2,

Fig. 4-Fig. 6: verschiedene Varianten für das erste Ausführungsbeispiel,

Fig. 7 eine Variante für das zweite Ausführungsbeispiel,

Fig. 8: eine schematische Darstellung eines dritten Ausführungsbeispiels eines erfindungsgemässen Rotationsfiltersystems,

Fig. 9: eine Variante für das dritte Ausführungsbeispiel,

Fig. 10: eine schematische Darstellung eines vierten Ausführungsbeispiels eines erfindungsgemässen Rotationsfiltersystems,

Fig. 11-Fig.12: zwei Varianten für das vierte Ausführungsbeispiel,

Fig. 13: eine schematische Darstellung eines Ausführungsbeispiels eines erfindungsgemässen Separationssystems für einen Bioreaktor, und

Fig. 14: eine Variante für das Ausführungsbeispiel aus Fig. 12.

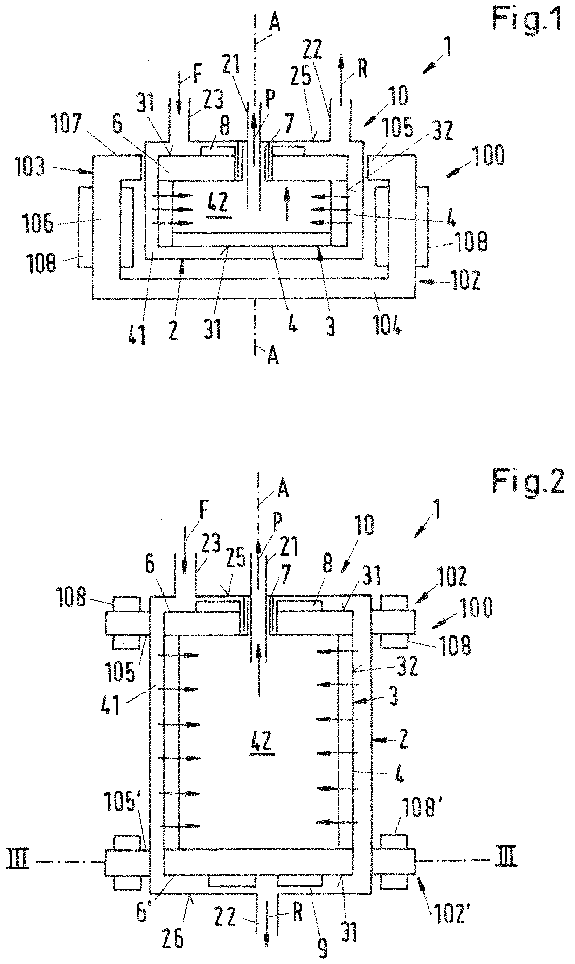

Fig. 1 zeigt in einer schematischen Darstellung ein erstes Ausführungsbeispiel eines erfindungsgemässen Rotationsfiltersystems, das gesamthaft mit dem Bezugszeichen 1 bezeichnet ist. Das Rotationsfiltersystem 1 dient zum Ausfiltern eines Filtrats aus einem Fluid, Das Fluid, welches dem Rotationsfiltersystem 1 zugeführt wird, ist durch den Pfeil F angedeutet und das Filtrat, das auch als Permeat bezeichnet wird, durch den Pfeil P. Der Pfeil R deutet ein Retentat an. Das Rotationsfiltersystem 1 umfasst eine Rotationsfiltervorrichtung 10 und eine Antriebsvorrichtung 100. Die Rotationsfiltervorrichtung 10 umfasst ein stationäres Filtergehäuse 2, das im Betriebszustand stationär ist, also nicht rotiert. In dem Filtergehäuse 2 ist eine um eine axiale Richtung A rotierbare Filtereinheit 3 vorgesehen, die vollständig von dem Filtergehäuse 2 umschlossen ist. Im Betriebszustand rotiert die Filtereinheit 3 im Inneren des stationären Filtergehäuses 2. Die Solldrehachse, um welche die Filtereinheit 3 rotieren soll, definiert die axiale Richtung A. Eine Richtung senkrecht zur axialen Richtung A wird als radiale Richtung bezeichnet.

Die Filtereinheit 3 ist im Wesentlichen zylindrisch ausgestaltet und weist drei Aussenflächen auf, nämlich zwei Stirnflächen 31, welche die Filtereinheit 3 bezüglich der axialen Richtung A begrenzen, und eine Umfangsfläche 32, welche die Filtereinheit 3 bezüglich der radialen Richtung begrenzt.

Das Filtergehäuse 2 umfasst einen Einlass 23 zum Einbringen des Fluids F in das Filtergehäuse 2, einen ersten Auslass 21 zum Abführen des Filtrats P aus dem Filtergehäuse 2 und einen zweiten Auslass 22 zum Abführen des Retentats R aus dem Filtergehäuse 2.

Die Filtereinheit 3 umfasst mindestens ein Filterelement 4, welches einen Fluidraum 41 im Filtergehäuse 2 von einem Filtratraum 42 im Filtergehäuse 2 abgrenzt. Der Filtratraum 42 ist radial innenliegend bezüglich des Filterelements 4 angeordnet, und der Fluidraum 41 ist radial aussenliegend bezüglich des Filterelements 4 angeordnet. Bei dem ersten Ausführungsbeispiel erstreckt sich das Filterelement 4 entlang der Umfangsfläche 32 der Filtereinheit 3 und entlang einer der beiden Stirnflächen 31 des Filtergehäuses 2, nämlich entlang der darstellungsgemäss unteren Stirnfläche 31 in Fig. 1.

Der erste Auslass 21 ist so ausgestaltet, dass er in Strömungsverbindung mit dem Filtratraum 42 ist, sodass das Filtrat P aus dem Filtratraum 42 durch den ersten Auslass 21 aus dem Filtergehäuse 2 abführbar ist.

Als Filterelement 4 ist jedes an sich bekannte Filterelement geeignet, wobei die Wahl eines geeigneten Filterelements 4 natürlich vom jeweiligen Anwendungsfall abhängt.

Im Betriebszustand rotiert das Filterelement 4 um die axiale Richtung A, beispielsweise mit einer Rotationsgeschwindigkeit im Bereich von 100 U/min (Umdrehungen pro Minute). Das Fluid F wird durch den Einlass 23 in den Fluidraum 41 eingebracht. Diejenigen Komponenten des Fluids F, für welche das Filterelement 4 durchlässig ist, gelangen durch das Filterelement 4 in den Filtratraum 42, wie dies die Pfeile ohne Bezugszeichen andeuten, und werden dann als Filtrat P aus dem Filtratraum 42 durch den ersten Auslass 21 abgeführt. Diejenigen Komponenten des Fluids F, welche das Filterelement 4 nicht durchdringen bzw. durchfliessen können, verbleiben im Fluidraum 41 und werden dann als Retentat R durch den zweiten Auslass 22 aus dem Filtergehäuse 2 abgeführt. Die Rotation des Filterelements 4 bewirkt, dass Ablagerungen auf oder in dem Filterelement 4 durch Zentrifugalkräfte nach aussen weggeschleudert werden, sodass ein Verstopfen oder ein Zusetzen des Filterelements 4 wirkungsvoll verhindert oder zumindest drastisch reduziert wird.

Da bei dem ersten Ausführungsbeispiel das Filterelement 4 an der Umfangsfläche 32 und an einer der Stirnflächen 31 vorgesehen ist, können die Komponenten, für welche das Filterelement 4 durchlässig ist, das Filterelement 4 sowohl in axialer Richtung A als auch in radialer Richtung durchdringen bzw. durchfliessen.

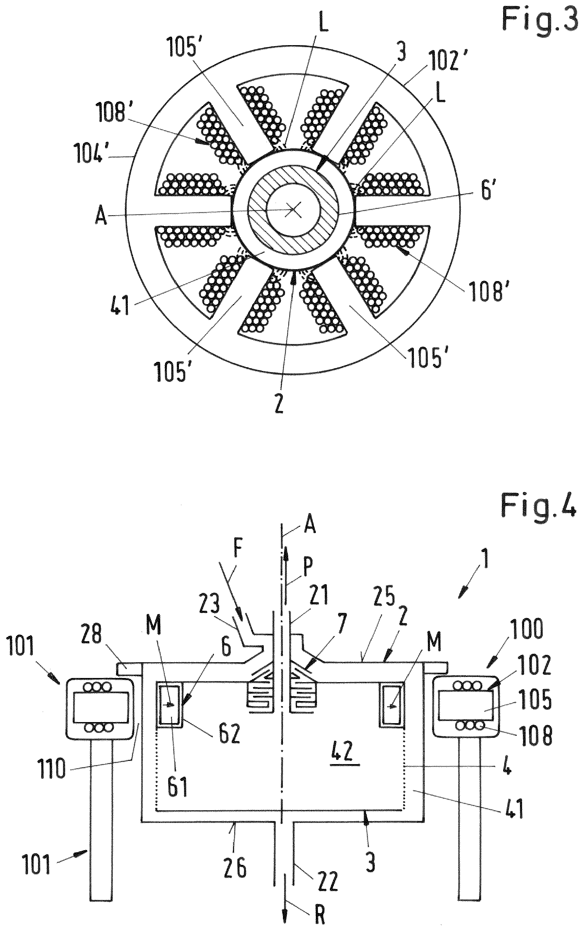

Die Filtereinheit 3 umfasst ferner einen magnetisch wirksamen Kern 6, der scheibenförmig oder ringförmig ausgestaltet ist. Der magnetisch wirksame Kern 6 ist an einer der beiden Stirnflächen 31 angeordnet, hier an der darstellungsgemäss oberen Stirnfläche 31 (gemäss der Darstellung in Fig. 1). Der magnetisch wirksame Kern 6 umfasst einen oder mehrere Permanentmagnete 61 (siehe z.B. Fig. 4), um die Filtereinheit 3 berührungslos magnetisch zur Rotation um die axiale Richtung A anzutreiben, und um die Filtereinheit 3 magnetisch, vorzugsweise berührungslos magnetisch zu lagern.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff verstanden, der eine Koerzitivfeldstärke, genauer gesagt eine Koerzitivfeldstärke der magnetischen Polarisation aufweist, die mehr als 10'000 A/m beträgt. Alle Permanentmagnete des magnetisch wirksamen Kerns 6 bestehen vorzugsweise aus Neodym-Eisen-Bor (NdFeB) oder aus Samarium-Cobalt (SmCo) Legierungen.

Der magnetisch wirksame Kern 6 kann ferner einen Rückschluss, beispielsweise einen ringförmigen Rückschluss umfassen, der zusammenhängend ausgestaltet ist und aus einem weichmagnetischen Material gefertigt ist. Beispielsweise ist jeder Permanentmagnet an der radial innenliegenden Seite des Rückschlusses angeordnet, sodass der Rückschluss den Permanentmagneten oder die Permanentmagnete umschliesst. Der Rückschluss führt den magnetischen Fluss und dient damit sowohl der Erzeugung des Drehmoments zum Antreiben der Rotation der Filtereinheit 3 als auch der magnetischen Lagerung der Filtereinheit 3. Geeignete weichmagnetische Materialien sind beispielsweise ferromagnetische oder ferrimagnetische Materialien, also insbesondere Eisen, Nickel-Eisen oder Silizium-Eisen.

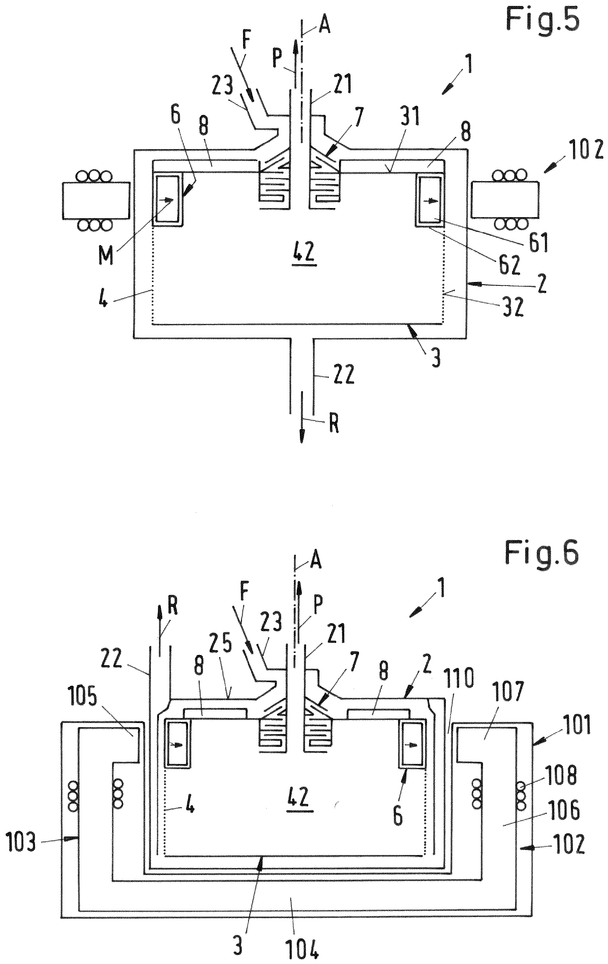

Die Antriebsvorrichtung 100 umfasst ein Antriebsgehäuse 101 (siehe z. B. Fig. 6), das in der schematischen Darstellung in Fig. 1 nicht dargestellt ist. In dem Antriebsgehäuse 101 ist ein Stator 102 zum Antreiben der Rotation der Filtereinheit 3 vorgesehen. Der Stator 102 ist als Lager- und Antriebsstator ausgestaltet, der mit dem magnetisch wirksamen Kern 6 der Filtereinheit 3 als elektromagnetischer Drehantrieb zusammenwirkt. Mit dem Lager- und Antriebsstator 102 ist die Filtereinheit 3 berührungslos magnetisch zur Rotation um eine Solldrehachse antreibbar und berührungslos magnetisch bezüglich des Stators 102 lagerbar. Dabei ist der elektromagnetische Drehantrieb als Innenläufer ausgestaltet, das heisst, der Stator 102 ist radial aussenliegend um das Filtergehäuse 2 herum angeordnet, sodass der Stator 102 den magnetisch wirksamen Kern 6 der Filtereinheit 3 umgibt. Die Solldrehachse bezeichnet diejenige Achse, um welche sich die Filtereinheit 3 im Betriebszustand dreht, wenn sich die Filtereinheit 3 bezüglich des Stators 102 in einer zentrierten und unverkippten Lage befindet.

Vorzugsweise umfasst das Antriebsgehäuse 101 eine zentral angeordnete Kavität 110 (Fig. 6), in welche das Filtergehäuse 2 einsetzbar ist. Dabei ist die Kavität 110 vorzugsweise so bemessen, dass der Abstand zwischen dem Stator 102 und dem magnetisch wirksamen Kern 6 in der radialen Richtung möglichst gering ist. Diese Ausgestaltung mit der Kavität 110 ist insbesondere auch dann vorteilhaft, wenn das Filtergehäuse 2 mit der darin angeordneten Filtereinheit 3 als Einmalteil für den Einmalgebrauch ausgestaltet ist. Das Filtergehäuse 2 kann dann in sehr einfacher Weise und vorzugsweise auch ohne die Verwendung eines Werkzeugs in die Antriebsvorrichtung 100 eingesetzt werden bzw. von der Antriebsvorrichtung 100 getrennt werden.

Der Stator 102 umfasst eine Mehrzahl von Spulenkernen 103, beispielsweise sechs Spulenkerne 103, welche über einen ring- oder scheibenförmigen Rückschluss 104 miteinander verbunden sind. Die Spulenkerne 103 und der Rückschluss 104 sind aus einem weichmagnetischen Material gefertigt. Jeder Spulenkerne 103 weist einen ausgeprägten Statorpol 105 auf, wobei der magnetisch wirksame Kern 6 derart zwischen den Statorpolen 105 angeordnet ist, dass die ausgeprägten Statorpole 105 dem magnetisch wirksamen Kern 6 radial aussenliegend gegenüberliegen und um den magnetisch wirksamen Kern 6 herum angeordnet sind.

Bei dem ersten Ausführungsbeispiel ist der elektromagnetische Drehantrieb als sogenannter Tempelmotor ausgestaltet. Jeder Spulenkern 103 hat jeweils einen Längsschenkel 106, welcher sich in axialer Richtung A erstreckt, sowie einen senkrecht zum Längsschenkel 106 angeordneten Querschenkel 107, welcher sich in einer radialen Richtung nach innen erstreckt. Der Querschenkel 107 ist jeweils an einem axialen Ende des zugehörigen Längsschenkels 106 angeordnet. Jeder Querschenkel 107 bildet einen der ausgeprägten Statorpole 105. Die Spulenkerne 103 sind äquidistant auf einer Kreislinie angeordnet, sodass die Querschenkel 107 den magnetisch wirksamen Kern 6 der Filtereinheit 3 umgeben, wenn das Filtergehäuse 2 in die Antriebsvorrichtung 100 eingesetzt ist. An jedem Längsschenkel 106 ist jeweils eine konzentrierte Wicklung 108 angeordnet, welche den jeweiligen Längsschenkel 106 umgibt. Es sind Ausführungsformen möglich, bei denen an jedem Längsschenkel 106 genau eine konzentrierte Wicklung 108 vorgesehen ist. In anderen Ausführungsformen können an jedem Längsschenkel 106 jeweils mehr als eine Wicklung, beispielsweise zwei konzentrierte Wicklungen vorgesehen sein.

Die Ausgestaltung als Tempelmotor ist eine besonders kompakte und gleichzeitig leistungsfähige Ausführungsform.

Mit den konzentrierten Wicklungen 108 werden die für den magnetischen Antrieb und die magnetische Lagerung der Filtereinheit 3 notwendigen elektromagnetischen Drehfelder erzeugt. Mit den konzentrierten Wicklungen 108 werden im Betriebszustand diejenigen elektromagnetischen Drehfelder erzeugt, mit welchen ein Drehmoment auf die Filtereinheit 3 bewirkt wird, und mit welchen eine beliebig einstellbare Querkraft in radialer Richtung auf die Filtereinheit 3 ausübbar ist, sodass die radiale Position der Filtereinheit 3, also ihre Position in der zur axialen Richtung A senkrechten radialen Ebene aktiv steuerbar bzw. regelbar ist.

Mit dem magnetisch wirksamen Kern 6 der Filtereinheit 3 werden die Komponenten der Filtereinheit 3 bezeichnet, welche für die Drehmomentbildung sowie für die Erzeugung der magnetischen Lagerkräfte magnetisch mit dem Stator 102 zusammenwirken, also beispielsweise der Permanentmagnet 61 bzw. die Permanentmagnete und der Rückschluss. Es versteht sich, dass der magnetisch wirksame Kern 6 drehfest mit dem Rest der Filtereinheit 3 verbunden ist.

Während des Betriebs des elektromagnetischen Drehantriebs wirkt der magnetisch wirksame Kern 6 der Filtereinheit 3 mit dem Stator 102 vorzugsweise nach dem Prinzip des lagerlosen Motors zusammen, bei welchem die Filtereinheit 3 als Rotor des elektromagnetischen Drehantriebs berührungslos magnetisch antreibbar und berührungslos magnetisch bezüglich des Stators 102 lagerbar ist, wobei kein separates magnetisches Lager vorgesehen ist. Dazu ist der Stator 102 als Lager- und Antriebsstator ausgestaltet, mit welchem die Filtereinheit 3 im Betriebszustand berührungslos magnetisch um die Solldrehachse antreibbar - also in Rotation versetzbar- und bezüglich des Stators 102 berührungslos magnetisch lagerbar ist. Dabei sind vorzugsweise drei Freiheitsgrade der Filtereinheit 3, nämlich ihre Position in der radialen Ebene und ihre Rotation, aktiv regelbar. Bezüglich seiner axialen Auslenkung aus der radialen Ebene in axialer Richtung A ist der magnetisch wirksame Kern 6 der Filtereinheit 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene ist der magnetisch wirksame Kern 6 der Filtereinheit 3 ebenfalls passiv magnetisch stabilisiert. Die Filtereinheit 3 ist also durch das Zusammenwirken des magnetisch wirksamen Kerns 6 mit den Spulenkernen 103 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist bezeichnet auch im Rahmen dieser Anmeldung eine aktive magnetische Lagerung eine solche, die aktiv steuer- bzw. regelbar ist, beispielsweise über die mit den konzentrierten Wicklungen 108 generierten elektromagnetischen Drehfelder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche die Filtereinheit 3 bei einer Auslenkung aus ihrer Soll-Position, also z. B. bei einer Verschiebung oder Auslenkung in axialer Richtung A oder bei einer Verkippung, wieder in ihre Soll-Position bringen.

Mit einer Radiallagerung oder einer radialen Lagerung wird eine Lagerung der Filtereinheit 3 bezeichnet, mit welcher die radiale Position der Filtereinheit 3 stabilisiert werden kann, also eine Lagerung, welche die Filtereinheit 3 in der radialen Ebene und damit bezüglich ihrer radialen Position lagert.

Mit einer Axiallagerung oder einer axialen Lagerung bzw. mit einer Axialstabilisierung oder einer axialen Stabilisierung wird eine Lagerung bzw. eine Stabilisierung der Filtereinheit 3 bezeichnet, mit welcher zum einen die Position der Filtereinheit 3 bezüglich der axialen Richtung A stabilisiert wird, und mit welcher zum anderen die Filtereinheit 3 gegen Verkippungen stabilisiert ist. Solche Verkippungen stellen zwei Freiheitsgrade dar und bezeichnen Auslenkungen, bei denen die momentane Drehachse der Filtereinheit 3 nicht mehr genau in die axiale Richtung A zeigt, sondern einen von Null verschiedenen Winkel mit der Solldrehachse einschliesst.

Beim lagerlosen Motor werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen 108 des Stators 102 erzeugten Drehfelder haben in der Regel eine Polpaarzahl, die sich um eins unterscheidet. Dabei werden mit dem Antriebsfeld auf den magnetisch wirksamen Kern 6 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 6 in der radialen Ebene erzeugen, mit welcher die Position des magnetisch wirksamen Kerns 6 in der radialen Ebene regelbar ist. Es ist also nicht möglich, den elektromagnetischen Fluss, der von den konzentrierten Wicklungen 108 generiert wird, aufzuteilen in einen (elektro-) magnetischen Fluss, der nur für den Antrieb der Rotation sorgt und einen (elektro-) magnetischen Fluss, der nur die magnetische Lagerung realisiert.

Diese Tatsache, dass die Antriebsfunktion und die Lagerfunktion nicht voneinander separierbar sind, ist es, welche dem Prinzip des lagerlosen Motors seinen Namen verleiht.

Zur Generierung des Antriebs- und des Steuerfelds ist es einerseits möglich, zwei unterschiedliche Wicklungssysteme zu verwenden, nämlich eines zur Erzeugung des Antriebsfelds mit einem Antriebsstrom und eines zur Erzeugung des Steuerfelds mit einem Steuerstrom. Andererseits ist es aber auch möglich, die Antriebs- und Lagerfunktion mit nur einem einzigen Wicklungssystem - wie in dem hier beschriebenen ersten Ausführungsbeispiel - zu generieren. Dies kann so realisiert werden, dass die von einer Kontrolleinrichtung jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 108 eingeprägt wird.

Wie dies in Fig. 1 dargestellt ist, ist im Innenraum des Filtergehäuses 2 eine berührungslose Dichtung 7 vorgesehen, welche zwischen der im Betriebszustand rotierenden Filtereinheit 3 und dem ersten Auslass 21 des Filtergehäuses 2 angeordnet ist. Der erste Auslass 21 ist im Zentrum des Filtergehäuses 2 vorgesehen, sodass er um die Solldrehachse herum angeordnet ist. Der erste Auslass 21 erstreckt sich bezüglich der axialen Richtung A durch die darstellungsgemäss (Fig. 1) obere Stirnfläche 31 der Filtereinheit 3 und durch das Zentrum des magnetisch wirksamen Kerns 6 hindurch bis in den Filtratraum 42, sodass das Filtrat P durch den ersten Auslass 21 aus dem Filtratraum 42 abführbar ist. Die berührungslose Dichtung 7 ist an der Durchführung des stationären Auslasses 21 in die im Betriebszustand rotierende Filtereinheit 3 vorgesehen. Vorzugsweise ist die berührungslose Dichtung 7 als Spaltdichtung und besonders bevorzugt als eine Labyrinthdichtung ausgestaltet. Die berührungslose Dichtung 7 soll den Leckagestrom vom Einlass 23 entlang des ersten Auslasses 21 in den Filtratraum 42 minimieren, also den Leckagestrom des Fluids, welcher das Filterelement 4 umgeht. Es ist nämlich möglich, dass ein Teil des Fluids F, ohne das Filterelement 4 zu passieren, vom Einlass 23 direkt in den Filtratraum 42 gelangt. Diese Leckage soll durch die berührungslose Dichtung 7 zumindest reduziert werden.

Dadurch, dass die berührungslose Dichtung 7 im Innenraum des Filtergehäuses 2 angeordnet ist, kann das Filtergehäuse 2 hermetisch dicht ausgestaltet werden, derart dass es die Filtereinheit 3 hermetisch umschliesst. Das Fluid F, das Filtrat P und das Retentat R können nur durch den Einlass 23 und die beiden Auslässe 21, 22 in das Filtergehäuse 2 einströmen bzw. aus dem Filtergehäuse 2 herausströmen. Ansonsten ist das Filtergehäuse 2 hermetisch dicht ausgestaltet. Insbesondere ist das stationäre Filtergehäuse 2 frei von dynamischen Dichtungen, welche das Filtergehäuse am Durchtritt einer rotierenden Welle abdichten müssten. Es ist somit keine dynamische Dichtung zwischen dem Innenraum des Filtergehäuses 2 und dem Aussenraum ausserhalb des Filtergehäuses 2 vorgesehen.

Vorzugsweise sind auf der Filtereinheit 3 und benachbart zu der berührungslosen Dichtung 7 Pumpenflügel 8 zum Fördern des Fluids vorgesehen. Die Pumpenflügel 8 sind auf der Stirnseite 31 angeordnet, durch welche der erste Auslass 21 hindurchführt. Gemäss der Darstellung in Fig. 1 ist dies die obere Stirnfläche 31. Die Pumpenflügel sind aussen an der Filtereinheit 3 angeordnet. Die Pumpenflügel 8 erstrecken sich in radialer Richtung und sind um die berührungslose Dichtung 7 herum angeordnet. Die Pumpenflügel 8 sind drehfest mit der Filtereinheit 3 verbunden und rotieren somit gemeinsam mit der Filtereinheit 3 um die axiale Richtung A.

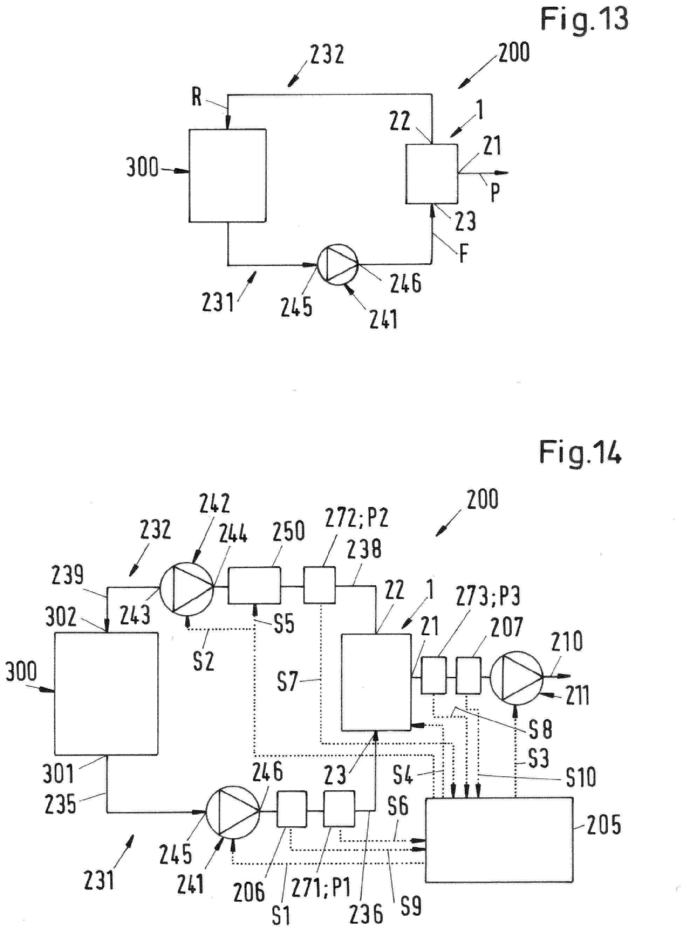

Die Pumpenflügel 8 dienen dazu, an der berührungslosen Dichtung 7 einen Druck aufzubauen und damit den Leckagestrom durch die berührungslose Dichtung 7 zu reduzieren. Je nach Ausgestaltung können die Pumpenflügel 8 zusätzlich auch dazu dienen, die Zirkulation des Fluid bzw. des Retentats anzutreiben. Ist das Rotationsfiltersystem 1 beispielsweise mit einem Bioreaktor verbunden (siehe z.B. Fig. 13), so können die Pumpenflügel 8 zumindest dazu beitragen, das Fluid aus dem Bioreaktor durch den Einlass 23 anzusaugen und das Retentat durch den zweiten Auslass 22 zum Bioreaktor zu rezirkulieren. Es sind aber auch solche Ausgestaltungen der Pumpenflügel 8 möglich, die nur lokal im Innenraum des Filtergehäuses 2, genauer gesagt, in der Umgebung der berührungslosen Dichtung 7, eine Druckerhöhung bewirken, um den Leckagestrom durch die berührungslose Dichtung 7 zu reduzieren.

Bei dem in Fig. 1 dargestellten ersten Ausführungsbeispiel weist das Filtergehäuse 2 eine Stirnfläche 25 auf, an welcher der Einlass 23, der erste Auslass 21 und der zweite Auslass 22 angeordnet sind. Somit sind alle Fluidöffnungen des Filtergehäuses 2, nämlich der Einlass 23 und die beiden Auslässe 21, 22, an der gleichen Stirnfläche 25 des Filtergehäuses 2 vorgesehen. Diese Ausgestaltung ist insbesondere vorteilhaft, wenn der elektromagnetische Drehantrieb als Innenläufer und als Tempelmotor ausgestaltet ist wie dies in Fig. 1 und auch in Fig. 6 dargestellt ist. Die zentral angeordnete Kavität 110 im Antriebsgehäuse 101 kann dann beispielsweise topf- oder becherförmig ausgestaltet sein, sodass das Filtergehäuse 2 in sehr einfacher Weise in die Antriebsvorrichtung 100 einsetzbar bzw. von der Antriebsvorrichtung 101 trennbar ist.

Fig. 2 zeigt in einer schematischen Darstellung ein zweites Ausführungsbeispiel eines erfindungsgemässen Rotationsfiltersystems 1.

Bei der folgenden Beschreibung des zweiten Ausführungsbeispiels wird nur auf die Unterschiede zu dem ersten Ausführungsbeispiel näher eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen des ersten Ausführungsbeispiels in gleicher Weise oder in sinngemäss gleicher Weise auch für das zweite Ausführungsbeispiel gelten.

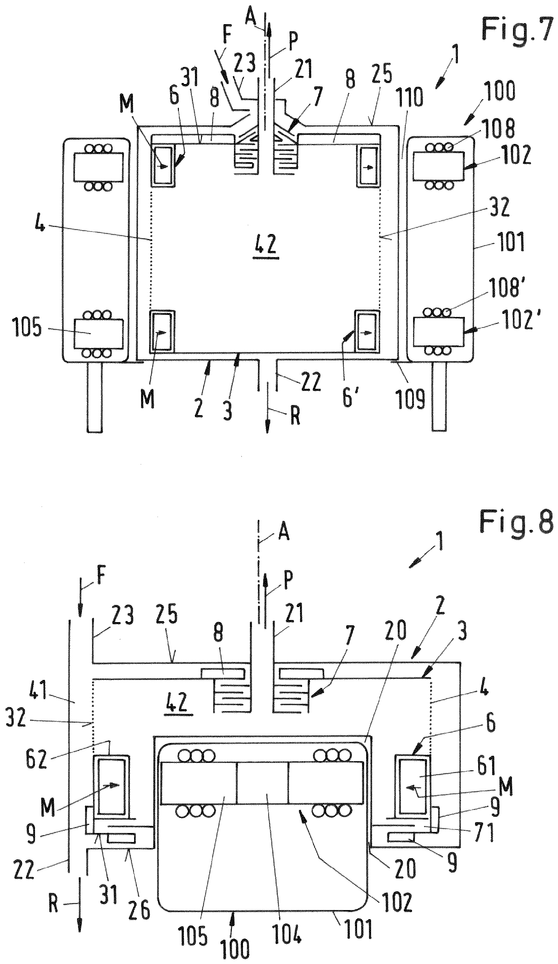

Bei dem zweiten Ausführungsbeispiel umfasst die Filtereinheit 3 zwei magnetisch wirksame Kerne 6, 6' von denen jeder scheiben- oder ringförmig ausgestaltet ist. Jeder der magnetisch wirksamen Kerne 6, 6' umfasst mindestens einen Permanentmagneten 61 (siehe z.B. Fig. 7). Die beiden magnetisch wirksamen Kerne 6, 6' sind bezüglich der axialen Richtung A beabstandet zueinander angeordnet. Vorzugsweise ist an jeder der beiden Stirnflächen 31 der Filtereinheit 3 jeweils ein magnetisch wirksamer Kern 6 bzw. 6' angeordnet, sodass der Abstand der beiden magnetisch wirksamen Kerne 31 voneinander möglichst gross ist. Die beiden magnetisch wirksamen Kerne 6, 6' sind koaxial und parallel zueinander angeordnet.

Im Antriebsgehäuse 101 der Antriebsvorrichtung 100 sind zwei Statoren 102, 102' zum Antreiben der Rotation der Filtereinheit 3 vorgesehen. In der schematischen Darstellung der Fig. 2 ist das Antriebsgehäuse 101 nicht dargestellt. Das Antriebsgehäuse 101 ist beispielsweise bei der in Fig. 7 dargestellten Variante dargestellt.

Jeder Stator 102, 102' ist als Lager- und Antriebsstator ausgestaltet, der mit jeweils einem der magnetischen Kerne 6, 6' der Filtereinheit 3 als elektromagnetischer Drehantrieb zusammenwirkt, sodass die Filtereinheit 3 berührungslos magnetisch zur Rotation antreibbar ist und bezüglich der der Statoren 102, 102' magnetisch, vorzugsweise berührungslos magnetisch lagerbar ist.

Vorzugsweise ist jeder der beiden elektromagnetischen Drehantriebe, die jeweils einen der Statoren 102, 102' und einen der magnetisch wirksamen Kerne 6, 6' umfassen, nach dem vorangehend beschriebenen Prinzip des lagerlosen Motors ausgestaltet.

Jeder der beiden elektromagnetischen Drehtriebe ist als Innenläufer ausgestaltet. Der eine der beiden Statoren 102 ist radial aussenliegend um den einen der magnetisch wirksamen Kerne 6 angeordnet, und der andere der beiden Statoren 102' ist radial aussenliegend um den anderen magnetisch wirksamen Kern 6' angeordnet.

Zum besseren Verständnis zeigt Fig. 3 noch einen Schnitt durch einen der Statoren 102' des zweiten Ausführungsbeispiels in einem Schnitt senkrecht zur axialen Richtung entlang der Schnittlinie III-III in Fig. 2. Fig 3 zeigt den in Fig. 2 darstellungsgemäss unteren Stator 102', wobei der andere Stator 102 in sinngemäss gleicher Weise ausgestaltet ist.

Beim zweiten Ausführungsbeispiel sind die elektromagnetischen Drehantriebe nicht als Tempelmotor ausgestaltet. Der ringförmige magnetisch wirksame Kern 6' der Filtereinheit 3 wird von dem radial aussenliegend angeordneten Stator 102' umgeben. Der Stator 102' umfasst eine Mehrzahl von ausgeprägten Statorpolen 105' - hier sechs Statorpole 105' -, die sich jeweils von dem radial aussenliegenden ringförmigen Rückschluss 104' in radialer Richtung nach innen auf das Filtergehäuse 2 mit dem darin angeordneten magnetisch wirksamen Kern 6' hin erstrecken. Jeder Statorpol 105' ist in der radialen Ebene angeordnet, in welcher der magnetisch wirksame Kern 6' im Betriebszustand gelagert und angetrieben wird. Während des Betriebs des elektromagnetischen Drehantriebs ist es die Soll-Position, dass der magnetisch wirksame Kern 6' zwischen den Statorpolen 105' zentriert ist.

Um die für den magnetischen Antrieb und die magnetische Lagerung des Filtergehäuses 3 notwendigen elektromagnetischen Drehfelder zu erzeugen, tragen die Statorpole 105' die konzentrierten Wicklungen 108'. Um jeden Statorpol 105' herum ist jeweils genau eine konzentrierte Wicklung 108' gewickelt ist, sodass auch jede konzentrierte Wicklung 108' in der radialen Ebene angeordnet ist. In anderen Ausführungsformen können auf jedem Statorpol 105'jeweils auch mehr als eine Wicklung, beispielsweise zwei Wicklungen vorgesehen sein.

In Fig. 3 sind auch noch Feldlinien L des elektromagnetischen Felds angedeutet, mit welchem die Filtereinheit 3 angetrieben bzw. gelagert wird.

Auch bei dem zweiten Ausführungsbeispiel ist das Filtergehäuse 2 hermetisch dicht ausgestaltet, derart dass es die Filtereinheit 3 hermetisch umschliesst. Das Fluid F, das Filtrat P und das Retentat R können nur durch den Einlass 23 und die beiden Auslässe 21, 22 in das Filtergehäuse 2 einströmen bzw. aus dem Filtergehäuse 2 herausströmen. Ansonsten ist das Filtergehäuse 2 hermetisch dicht ausgestaltet.

Bei dem zweiten Ausführungsbeispiel sind an der darstellungsgemäss (Fig. 2) oberen Stirnfläche 25 des Filtergehäuses 2 der Einlass 23 für das Fluid F und der erste Auslass 21 für das Abführen des Filtrats P aus dem Filtratraum 42 vorgesehen. Der zweite Auslass 22 zum Abführen des Retentats R aus dem Filtergehäuse 2 ist an der anderen Stirnfläche 26 des Filtergehäuses 2 vorgesehen, also der darstellungsgemäss unteren Stirnfläche 26 in Fig. 2. Der zweite Auslass 22 ist zentral in der Mitte der anderen Stirnfläche 26 angeordnet.

Da bei dem zweiten Ausführungsbeispiel an beiden Stirnflächen 31 der Filtereinheit 3 jeweils einer der beiden magnetischen Kerne 6, 6' angeordnet ist, erstreckt sich das Filterelement 4 nur entlang der Umfangsfläche 32 der Filtereinheit 3. Diejenigen Komponenten, für welche das Filterelement 4 durchlässig ist, durchdringen bzw. durchfliessen das Filterelement 4 nur in radialer Richtung von aussen nach innen, wie dies die Pfeile ohne Bezugszeichen in Fig. 2 andeuten.

Als eine weitere Option, die natürlich auch bei dem ersten Ausführungsbeispiel vorgesehen sein kann, sind an mindestens einer Aussenseite 31, 32 der Filtereinheit 3 eine Mehrzahl von Schaufeln 9 zum Erzeugen eines Transmembrandrucks über das Filterelement 4 vorgesehen. Die Schaufeln 9 sind zur Rotation um die axiale Richtung A ausgestaltet und drehfest mit der Filtereinheit 3 verbunden. Wie dies Fig. 2 zeigt, können die Schaufeln 9 beispielsweise aussen auf der darstellungsgemäss unteren Stirnfläche 31 der Filtereinheit 3 angeordnet sein. Jede Schaufel 9 erstreckt sich in radialer Richtung nach aussen.

Die Ausgestaltung des Rotationsfiltersystems 1 mit zwei elektromagnetischen Drehantrieben, also mit den beiden Lager- und Antriebsstatoren 102, 102' und mit den beiden magnetisch wirksamen Kernen 6, 6' hat den Vorteil, dass einerseits ein stärkeres Drehmoment zum Antreiben der Rotation der Filtereinheit 3 erzeugt werden kann, und dass andererseits die magnetische Lagerung der Filtereinheit 3 stabiler und stärker belastbar ist.

Vorzugsweise sind die beiden elektromagnetischen Drehantriebe zumindest im Wesentlichen identisch ausgestaltet. Es sei jedoch betont, dass die beiden elektromagnetischen Drehantriebe, auch wenn sie zumindest im Wesentlichen identisch ausgestaltet sind, im Betriebszustand des Rotationsfiltersystems 1 nicht in identischer Weise betrieben werden müssen. So ist es beispielsweise möglich, das Drehmoment, welches die Rotation der Filtereinheit 3 antreibt, nur mit einem der beiden elektromagnetischen Drehantriebe zu generieren, und den anderen der beiden elektromagnetischen Drehantriebe nur zur Erzeugung von Lagerkräften für die berührungslos magnetische Lagerung der Filtereinheit 3 zu verwenden, sodass also nur an einem der beiden magnetisch wirksamen Kerne 6, 6' ein Drehmoment generiert wird, welches die Rotation der Filtereinheit 3 antreibt. Die Lagerkräfte werden vorzugsweise an beiden magnetisch wirksamen Kernen generiert. Natürlich ist es auch möglich, dass mit beiden elektromagnetischen Drehantrieben jeweils ein Drehmoment generiert wird, welches die Rotation der Filtereinheit 3 antreibt, dass also an beiden magnetisch wirksamen Kernen 6, 6' ein Drehmoment generiert wird. Es versteht sich, dass in diesem Falle die an den beiden magnetisch wirksamen Kernen 6, 6' eingeprägten Drehmomente für den Antrieb der Filtereinheit 3 betragsmässig gleich gross oder auch verschieden sein können.

Anhand der Fig. 4 bis Fig. 6 werden nun einige Varianten erläutert, die insbesondere für das erste Ausführungsbeispiel (Fig. 1) geeignet sind, also für solche Ausführungsformen, bei welchen nur ein magnetisch wirksamer Kern 6 und nur ein Stator 102 und somit nur ein elektromagnetischer Drehantrieb vorgesehen ist.

Bei der in Fig. 4 dargestellten Variante ist der Stator 102 ringförmig ausgestaltet, nämlich in sinngemäss gleicher Weise, wie es im Zusammenhang mit dem zweiten Ausführungsbeispiel erläutert wurde und in Fig. 3 dargestellt ist. Die konzentrierten Wicklungen 108 sind alle in der radialen Ebene angeordnet, in welcher der magnetisch Wirksame Kern 6 gelagert und angetrieben wird. Der Stator 102 ist in dem Antriebsgehäuse 101 angeordnet, das als hermetisch dichtes Antriebsgehäuse 101 ausgestaltet ist.

Die zentral angeordnete Kavität 110 des Antriebsgehäuses 101, in welche das Filtergehäuse 2 einsetzbar ist, ist als durchgängig zentrale Öffnung ausgestaltet, welche sich in axialer Richtung A vollständig durch das Antriebsgehäuse 101 hindurch erstreckt. Alternativ kann die Kavität 110 auch durch einen Boden in axialer Richtung begrenzt sein. Das Filtergehäuse 2 umfasst ein Stützelement 28, mit welchem sich das Filtergehäuse 2 auf dem Antriebsgehäuse 101 abstützt. Das Stützelement 28 ist beispielsweise als radial aussenliegender Flansch ausgestaltet, welcher das Filtergehäuse 2 an seinem äusseren Umfang umgibt.

Der magnetisch wirksame Kern 6 der Filtereinheit 3 umfasst einen ringförmigen Permanentmagneten 61, der an der darstellungsgemäss (Fig. 4) oberen Stirnfläche 31 der Filtereinheit 3 angeordnet ist. Der Aussendurchmesser des ringförmigen Permanentmagneten 61 entspricht im Wesentlichen dem Aussendurchmesser der Filtereinheit 3. Der Permanentmagnet 61 ist vorzugsweise diametral magnetisiert, so wie dies die Pfeile mit dem Bezugszeichen M in Fig. 4 anzeigen.

Vorzugsweise ist ferner eine Ummantelung 62 vorgesehen, mit welcher der magnetisch wirksame Kern 6 umschlossen und vorzugsweise hermetisch eingekapselt ist, sodass der magnetisch wirksame Kern 6 nicht in Kontakt mit dem Fluid F, dem Filtrat P oder dem Retentat R kommt.

Bei der in Fig. 4 dargestellten Variante ist der zweite Auslass 22, durch welchen das Retentat R aus dem Filtergehäuse 2 abgeführt wird, in sinngemäss gleicher Weise wir bei dem zweiten Ausführungsbeispiel (Fig. 2) in der darstellungsgemäss (Fig. 4) unteren Stirnfläche 26 des Filtergehäuses 2 und vorzugsweise im Zentrum diese Stirnfläche 26 angeordnet.

Der erste Auslass 21, durch welchen das Filtrat P aus dem Filtratraum 42 abgeführt wird, ist im Zentrum der darstellungsgemäss (Fig. 4) oberen Stirnfläche 25 des Filtergehäuses 2 angeordnet. Der Einlass 23 für das Fluid F ist konzentrisch um den ersten Auslass 21 herum angeordnet.

Die berührungslose Dichtung 7 ist als Labyrinthdichtung ausgestaltet.

Bei der in Fig. 5 dargestellten Variante sind auf der darstellungsgemäss oberen Stirnfläche 31 der Filtereinheit die Pumpenflügel 8 zum Fördern des Fluids vorgesehen. Dabei sind die Pumpenflügel 8 mit einer grossen Erstreckung in radialer Richtung ausgestaltet, beispielsweise derart, dass sie sich bezüglich der radialen Richtung bis an die Umfangsfläche 32 der Filtereinheit 3 erstrecken. Zum einen dienen die Pumpenflügel 8 dazu, die Leckage durch die berührungslose Dichtung 7 zu reduzieren, und zum anderen erzeugen die Pumpenflügel 8 einen ausreichenden Druck und einen ausreichenden Fluss, um das Fluid durch das Rotationsfiltersystem 1 zu fördern.

Ist das Rotationsfiltersystem 1 beispielsweise mit einem Bioreaktor verbunden (siehe z.B. Fig. 13), so können die Pumpenflügel 8 zumindest dazu beitragen, das Fluid F aus dem Bioreaktor durch den Einlass 23 anzusaugen und das Retentat R durch den zweiten Auslass 22 zum Bioreaktor zu rezirkulieren. Je nach Ausgestaltung können die Pumpenflügel 8 auch die einzige Pumpvorrichtung zum Zirkulieren des Fluids F bzw. des Retentats R sein.

Die in Fig. 6 dargestellte Variante ist insbesondere für solche Ausführungsformen vorteilhaft, bei denen der elektromagnetische Drehantrieb als Tempelmotor ausgestaltet ist. Bei der in Fig. 6 dargestellten Variante sind sowohl der Einlass 23 als auch die beiden Auslässe 21, 22 in dergleichen Stirnfläche 25, nämlich in der darstellungsgemäss oberen Stirnfläche 25 des Filtergehäuses 2 angeordnet. Dabei ist der Einlass 23 in gleicher Weise wie bei der in Fig. 5 dargestellten Variante konzentrisch um den zentral angeordneten ersten Auslass 21 herum angeordnet. Der zweite Auslass 22 ist an der Peripherie der Stirnfläche 25 des Filtergehäuses 2 angeordnet.

Dadurch, dass der Einlass 23 und die beiden Auslässe 21, 22 in der gleichen Stirnfläche 25 des Filtergehäuses 2 angeordnet sind, kann die Kavität 110 in dem Antriebsgehäuse 101 becherförmig oder topfförmig ausgestaltet werden, sodass das Filtergehäuse 2 in sehr einfacher Weise in die Antriebsvorrichtung 100 einsetzbar ist.

In Fig. 7 ist eine Variante für das zweite Ausführungsbeispiel (Fig. 2) gezeigt, also für solche Ausführungsformen, bei welchen jeweils zwei magnetisch wirksame Kerne 6, 6' und zwei Statoren 102, 102' und somit zwei elektromagnetische Drehantriebe vorgesehen sind.

Bei der in Fig. 7 dargestellten Variante ist das Antriebsgehäuse 101, in welchem die beiden Statoren 102, 102' angeordnet sind, als im Wesentlichen ringförmiges Antriebsgehäuse 101 ausgestaltet. Die zentral angeordnete Kavität 110 des Antriebsgehäuses 101, in welche das Filtergehäuse 2 einsetzbar ist, ist als durchgängig zentrale Öffnung ausgestaltet, welche sich in axialer Richtung A vollständig durch das Antriebsgehäuse 101 hindurch erstreckt. Das Antriebsgehäuse 101 umfasst ein Abstützung 109, auf welcher sich das Filtergehäuse 2 abstützen kann, wenn es in die Antriebsvorrichtung 100 eingesetzt ist. Die Abstützung 109 ist beispielsweise als ein ringförmiger Vorsprung ausgestaltet, der sich nach innen in die Kavität 110 erstreckt und auf welcher sich das Filtergehäuse 2 abstützen kann.

Vorzugsweise ist das Antriebsgehäuse 101 hermetisch dicht ausgestaltet.

Der erste Auslass 21, durch welchen das Filtrat P aus dem Filtratraum 42 abgeführt wird, ist im Zentrum der darstellungsgemäss (Fig. 7) oberen Stirnfläche 25 des Filtergehäuses 2 angeordnet. Der Einlass 23 für das Fluid F ist konzentrisch um den ersten Auslass 21 herum angeordnet.

Die berührungslose Dichtung 7 ist als Labyrinthdichtung ausgestaltet.

Bei der in Fig. 7 dargestellten Variante sind auf der darstellungsgemäss oberen Stirnfläche 31 der Filtereinheit 3 die Pumpenflügel 8 zum Fördern des Fluids vorgesehen. Dabei sind die Pumpenflügel 8 mit einer grossen Erstreckung in radialer Richtung ausgestaltet, beispielsweise derart, dass sie sich bezüglich der radialen Richtung bis an die Umfangsfläche 32 der Filtereinheit 3 erstrecken. Zum einen dienen die Pumpenflügel 8 dazu, die Leckage durch die berührungslose Dichtung 7 zu reduzieren, und zum anderen erzeugen die Pumpenflügel 8 einen ausreichenden Druck und einen ausreichenden Fluss, um das Fluid durch das Rotationsfiltersystem 1 zu fördern.

Ist das Rotationsfiltersystem 1 beispielsweise mit einem Bioreaktor verbunden (siehe z.B. Fig. 13), so können die Pumpenflügel 8 zumindest dazu beitragen, das Fluid F aus dem Bioreaktor durch den Einlass 23 anzusaugen und das Retentat R durch den zweiten Auslass 22 zum Bioreaktor zu rezirkulieren. Je nach Ausgestaltung können die Pumpenflügel 8 auch die einzige Pumpvorrichtung zum Zirkulieren des Fluids F bzw. des Retentats R sein.

Fig. 8 zeigt in einer schematischen Darstellung ein drittes Ausführungsbeispiel eines erfindungsgemässen Rotationsfiltersystems 1.

Bei der folgenden Beschreibung des dritten Ausführungsbeispiels wird nur auf die Unterschiede zu dem ersten und dem zweiten Ausführungsbeispiel sowie ihrer Varianten näher eingegangen. Gleiche Teile oder von der Funktion her gleichwertige Teile des dritten Ausführungsbeispiels sind mit den gleichen Bezugszeichen bezeichnet wie bei dem ersten und dem zweiten Ausführungsbeispiel und ihren Varianten. Insbesondere haben die Bezugszeichen die gleiche Bedeutung wie sie bereits im Zusammenhang mit dem ersten und dem zweiten Ausführungsbeispielen sowie ihren Varianten erläutert sind. Es versteht sich, dass alle vorangehenden Erläuterungen des ersten und des zweiten Ausführungsbeispiels sowie ihrer Varianten in gleicher Weise oder in sinngemäss gleicher Weise auch für das dritte Ausführungsbeispiel gelten.

Das dritte Ausführungsbeispiel ist in sinngemäss gleicher Weise wie das erste Ausführungsbeispiel eine Ausführungsform, bei welchen nur ein magnetisch wirksamer Kern 6 und nur ein Stator 102 und somit nur ein elektromagnetischer Drehantrieb vorgesehen ist. Im Unterschied zu dem ersten Ausführungsbeispiel ist bei dem dritten Ausführungsbeispiel der elektromagnetische Drehantrieb, welcher den magnetisch wirksamen Kern 6 und den Stator 102 umfasst, als Aussenläufer ausgestaltet. Bei der Ausgestaltung als Aussenläufer ist der Stator 102 radial innenliegend im Filtergehäuse 2 angeordnet, sodass der magnetisch wirksame Kern 6 der Filtereinheit 3 den im Antriebsgehäuse 101 angeordneten Stator 102 umgibt.

Dazu umfasst das stationäre Filtergehäuse 2 eine zentrale Ausnehmung 20, in welche das Antriebsgehäuse 101 einsetzbar ist, sodass der Stator 102 innerhalb des magnetisch wirksamen Kerns 6 angeordnet ist. Bezüglich der axialen Richtung A sind die Statorpole 105 auf der gleichen Höhe angeordnet wie der magnetisch wirksame Kern 6, wenn das Antriebsgehäuse 101 in die zentrale Ausnehmung 20 des Filtergehäuses 2 eingesetzt ist

Die in Fig. 8 gezeigte Ausgestaltung mit der zentralen Ausnehmung 20, in welche die Antriebsvorrichtung 100 eingesetzt werden kann, ist insbesondere auch dann vorteilhaft, wenn das Filtergehäuse 2 mit der darin angeordneten Filtereinheit 3 als Einmalteil für den Einmalgebrauch ausgestaltet ist. Die Antriebsvorrichtung 100 kann dann in sehr einfacher Weise und vorzugsweise auch ohne die Verwendung eines Werkzeugs in das Filtergehäuse 2 eingesetzt werden bzw. von dem Filtergehäuse 2 getrennt werden.

Der magnetisch wirksame Kern 6 der Filtereinheit 3 umfasst vorzugsweise den ringförmigen Permanentmagneten 61, der bei dem dritten Ausführungsbeispiel vorzugsweise an der darstellungsgemäss (Fig. 8) unteren Stirnfläche 31 der Filtereinheit 3 angeordnet ist. Der Aussendurchmesser des ringförmigen Permanentmagneten 61 entspricht im Wesentlichen dem Aussendurchmesser der Filtereinheit 3. Der Permanentmagnet 61 ist vorzugsweise radial von aussen nach innen magnetisiert, so wie dies die Pfeile mit dem Bezugszeichen M in Fig. 8 anzeigen.

Im Bereich der darstellungsgemäss (Fig. 8) unteren Stirnfläche 31 ist vorzugsweise eine zusätzliche Dichtung 71 zum Abdichten zwischen der im Betriebszustand rotierenden Filtereinheit 3 und dem stationären Filtergehäuse 2 vorgesehen. Auch die zusätzliche Dichtung 71 ist als berührungslose Dichtung ausgestaltet, vorzugsweise als Spaltdichtung und besonders bevorzugt als Labyrinthdichtung. Die zusätzliche Dichtung 71 reduziert den Leckagestrom des Fluids direkt aus dem Fluidraum 41 in den Filtratraum 42, also den Leckagestrom des Fluids, welcher das Filterelement 4 umgeht. Die zusätzliche Dichtung 71 ist im Innenraum des Filtergehäuses 2 angeordnet, sodass auch beim dritten Ausführungsbeispiels das Filtergehäuse 2 hermetisch dicht ausgestaltet werden kann.

Der Einlass 23 für das Fluid F ist an der Peripherie der Stirnfläche 25 des Filtergehäuses 2 angeordnet. An der gleichen Stirnfläche 25 des Filtergehäuses 2 ist auch der erste Auslass 21 vorgesehen, durch welchen das Filtrat P aus dem Filtratraum 42 abgeführt werden kann. Der erste Auslass 21 ist zentral in der Mitte der Stirnfläche 25 des Filtergehäuses 2 angeordnet. Der zweite Auslass 22 zum Abführen des Retentats R ist an der Peripherie der anderen Stirnfläche 26 des Filtergehäuses 2 angeordnet.

Benachbart zu der berührungslosen Dichtung 7 können optional auch bei dem dritten Ausführungsbeispiel die Pumpenflügel 8 vorgesehen sein, um an der berührungslosen Dichtung 7 einen Druck aufzubauen und damit den Leckagestrom durch die berührungslose Dichtung 7 zu reduzieren.

Als eine weitere Option, sind an mindestens einer Aussenseite 31, 32 der Filtereinheit 3 die Mehrzahl von Schaufeln 9 zum Erzeugen eines Transmembrandrucks über das Filterelement 4 vorgesehen. Die Schaufeln 9 sind zur Rotation um die axiale Richtung A ausgestaltet und drehfest mit der Filtereinheit 3 verbunden. Wie dies Fig. 8 zeigt, können die Schaufeln 9 an der Umfangsfläche 32 der Filtereinheit 3 angeordnet sein und sich jeweils in axialer Richtung A erstrecken, und/oder die Schaufeln 9 können aussen auf der darstellungsgemäss unteren Stirnfläche 31 der Filtereinheit 3 angeordnet sein, benachbart zu der zusätzlichen Dichtung 71. Jede der auf der Stirnfläche 31 angeordnete Schaufel 9 erstreckt sich in radialer Richtung nach aussen.

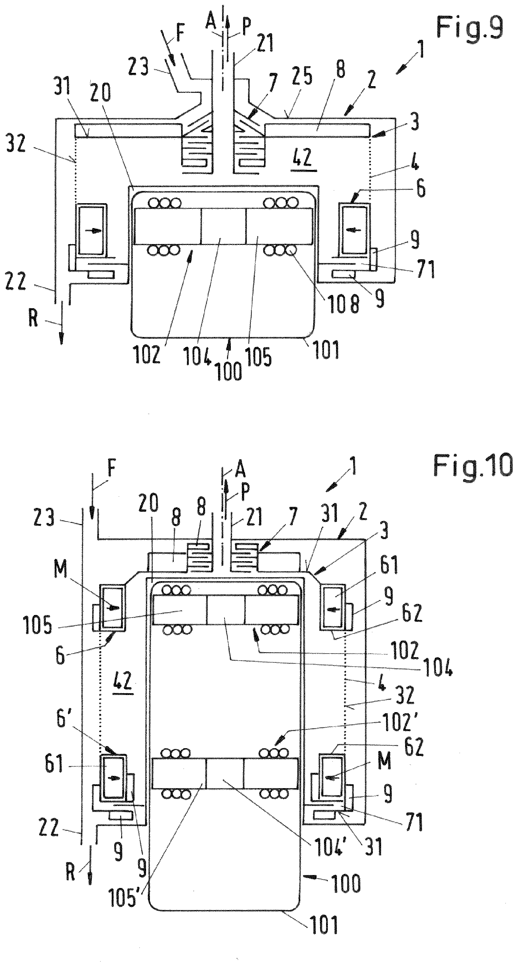

Fig. 9 zeigt eine Variante des dritten Ausführungsbeispiels, bei welcher auf der darstellungsgemäss oberen Stirnfläche 31 der Filtereinheit die Pumpenflügel 8 zum Fördern des Fluids vorgesehen sind. Dabei sind die Pumpenflügel 8 mit einer grossen Erstreckung in radialer Richtung ausgestaltet sodass sie eine gute Pumpwirkung erzeugen können. Beispielsweise sind die Pumpenflügel 8 so ausgestaltet, dass sie sich bezüglich der radialen Richtung bis an die Umfangsfläche 32 der Filtereinheit 3 erstrecken. Zum einen dienen die Pumpenflügel 8 dazu, die Leckage durch die berührungslose Dichtung 7 zu reduzieren, und zum anderen erzeugen die Pumpenflügel 8 einen ausreichenden Druck und einen ausreichenden Fluss, um das Fluid durch das Rotationsfiltersystem 1 zu fördern.

Ist das Rotationsfiltersystem 1 beispielsweise mit einem Bioreaktor verbunden (siehe z.B. Fig. 13), so können die Pumpenflügel 8 zumindest dazu beitragen, das Fluid F aus dem Bioreaktor durch den Einlass 23 anzusaugen und das Retentat R durch den zweiten Auslass 22 zum Bioreaktor zu rezirkulieren. Je nach Ausgestaltung können die Pumpenflügel 8 auch die einzige Pumpvorrichtung zum Zirkulieren des Fluids F bzw. des Retentats R sein.

Bei der in Fig. 9 dargestellten Variante ist der erste Auslass 21, durch welchen das Filtrat P aus dem Filtratraum 42 abgeführt wird, im Zentrum der darstellungsgemäss (Fig. 9) oberen Stirnfläche 25 des Filtergehäuses 2 angeordnet. Der Einlass 23 für das Fluid F ist konzentrisch um den ersten Auslass 21 herum angeordnet.

Fig. 10 zeigt in einer schematischen Darstellung ein viertes Ausführungsbeispiel eines erfindungsgemässen Rotationsfiltersystems 1.

Bei der folgenden Beschreibung des vierten Ausführungsbeispiels wird nur auf die Unterschiede zu den vorangehend beschriebenen Ausführungsbeispielen sowie ihrer Varianten näher eingegangen. Gleiche Teile oder von der Funktion her gleichwertige Teile des vierten Ausführungsbeispiels sind mit den gleichen Bezugszeichen bezeichnet wie bei den vorangehend beschriebenen Ausführungsbeispielen und ihren Varianten. Insbesondere haben die Bezugszeichen die gleiche Bedeutung wie sie bereits im Zusammenhang mit den vorangehend beschriebenen Ausführungsbeispielen sowie ihren Varianten erläutert sind. Es versteht sich, dass alle vorangehenden Erläuterungen bezüglich der ersten drei Ausführungsbeispiele sowie ihrer Varianten in gleicher Weise oder in sinngemäss gleicher Weise auch für das vierten Ausführungsbeispiel gelten.

Bei dem vierten Ausführungsbeispiel sind in sinngemäss gleicher Weise wie bei dem zweiten Ausführungsbeispiel zwei elektromagnetische Drehantriebe vorgesehen. Diese sind bei dem vierten Ausführungsbeispiel jedoch beide als Aussenläufer ausgestaltet.

Dazu umfasst das stationäre Filtergehäuse 2 die zentrale Ausnehmung 20, in welche das Antriebsgehäuse 101 einsetzbar ist,

Bei dem vierten Ausführungsbeispiel umfasst die Filtereinheit 3 zwei magnetisch wirksame Kerne 6, 6', von denen jeder ringförmig ausgestaltet ist, und die optional jeweils die Ummantelung 62 aufweisen, welche den jeweiligen magnetisch wirksamen Kern 6, 6' umkapselt. Jeder der magnetisch wirksamen Kerne 6, 6' umfasst mindestens einen Permanentmagneten 61. Die beiden magnetisch wirksamen Kerne 6, 6' sind bezüglich der axialen Richtung A beabstandet zueinander angeordnet. Vorzugsweise ist an jeder der beiden Stirnflächen 31 der Filtereinheit 3 jeweils ein magnetisch wirksamer Kern 6 bzw. 6' angeordnet, sodass der Abstand der beiden magnetisch wirksamen Kerne 6, 6' voneinander möglichst gross ist. Die beiden magnetisch wirksamen Kerne 6, 6' sind koaxial und parallel zueinander angeordnet.

Im Antriebsgehäuse 101 der Antriebsvorrichtung 100 sind zwei Statoren 102, 102' zum Antreiben der Rotation der Filtereinheit 3 vorgesehen.

Jeder Stator 102, 102' ist als Lager- und Antriebsstator ausgestaltet, der mit jeweils einem der magnetischen Kerne 6, 6' der Filtereinheit 3 als elektromagnetischer Drehantrieb zusammenwirkt, sodass die Filtereinheit 3 berührungslos magnetisch zur Rotation antreibbar ist und bezüglich der der Statoren 102, 102' magnetisch, vorzugsweise berührungslos magnetisch lagerbar ist.

Jeder der beiden elektromagnetischen Drehantriebe ist als Aussenläufer ausgestaltet. Der eine der beiden magnetisch wirksamen Kerne 6 ist radial aussenliegend um den einen der Statoren 102 angeordnet, und der andere der beiden magnetisch wirksamen Kerne 6' ist radial aussenliegend um den anderen der beiden Statoren 102' angeordnet.

Jeder Stator 102, 102' ist also innerhalb eines der magnetisch wirksamen Kerne 6, 6. angeordnet. Bezüglich der axialen Richtung A sind die Statorpole 105 bzw. 105' auf der gleichen Höhe angeordnet wie der magnetisch wirksame Kern 6 oder 6', welcher diese Statorpole 105 oder 105' umgibt, wenn das Antriebsgehäuse 101 in die zentrale Ausnehmung 20 des Filtergehäuses 2 eingesetzt ist. Die Statorpole 105 bzw. 105' erstrecken sich jeweils von dem radial innenliegenden ringförmigen Rückschluss 104, 104' sternförmig in radialer Richtung nach aussen.

Optional sind auch bei dem vierten Ausführungsbeispiel Pumpenflügel 8 und/oder Schaufeln 9 zum Erzeugen eines Transmembrandrucks vorgesehen.

Pumpenflügel 8 können benachbart zu der berührungslosen Dichtung 7 und/oder auf der berührungslosen Dichtung 7 vorgesehen sein. Die Schaufeln 9 können an einer oder mehreren der folgenden Stellen vorgesehen sein:

- radial aussenliegend auf der Umfangsfläche 32 der Filtereinheit 3 auf Höhe (bezüglich der axialen Richtung A) des darstellungsgemäss (Fig. 10) oberen magnetisch wirksamen Kerns 6,

- radial aussenliegend auf der Umfangsfläche 32 der Filtereinheit 3 auf Höhe (bezüglich der axialen Richtung A) des darstellungsgemäss (Fig. 10) unteren magnetisch wirksamen Kerns 6',

- radial innenliegend an dem darstellungsgemäss (Fig. 10) unteren magnetisch wirksamen Kerns 6' und damit benachbart zu der zusätzlichen Dichtung 71,

- aussen auf der darstellungsgemäss (Fig. 10) unteren Stirnfläche 31 der Filtereinheit 3 benachbart zu der zusätzlichen Dichtung 71.

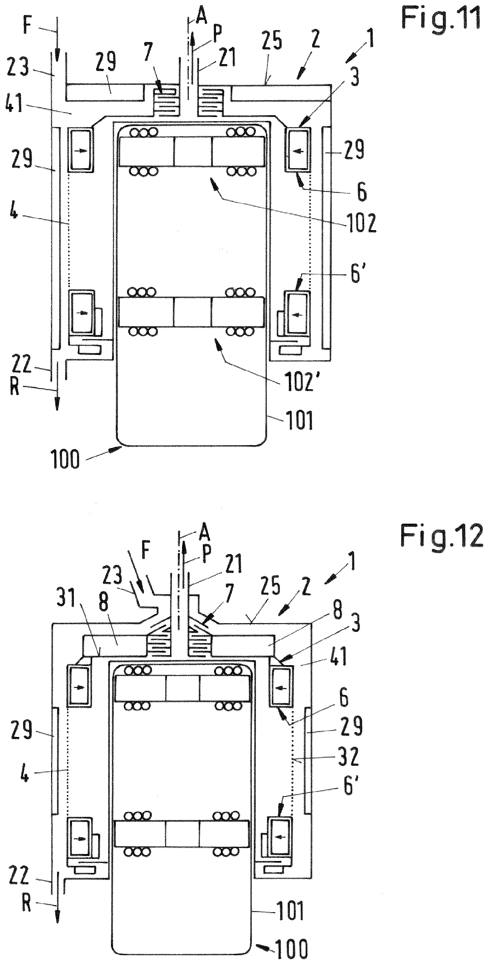

Die Fig. 11 und Fig. 12 zeigen Varianten des vierten Ausführungsbeispiels, wobei die Varianten auch für das dritte Ausführungsbeispiel verwendet werden können.

Bei der in Fig. 11 dargestellten Variante sind im stationären Filtergehäuse 2 stationäre Statorschaufeln 29 zur Erzeugung einer zusätzlichen rotativen Strömung relativ zu dem im Betriebszustand rotierenden Filterelement 4 vorgesehen. Die Statorschaufeln 29 sind innen an der Wandung vorgesehen, welche das Filtergehäuse 2 begrenzt und reichen in den Fluidraum 41 des Filtergehäuses 2 hinein. Die Statorschaufeln 29 können an der radial aussenliegenden Wandung des Filtergehäuses 2 angeordnet sein mit einer Längserstreckung in axialer Richtung A. Alternativ oder zusätzlich können die Statorschaufeln 29 auch innen an der Stirnfläche 25 des Filtergehäuses 2, darstellungsgemäss (Fig. 11) an der oberen Stirnfläche 25, vorgesehen sein mit einer Längserstreckung in radialer Richtung. Die Funktion der stationären Statorschaufeln 29 ist es, Stauzonen für das Fluid F zu bilden, das zwischen den stationären Statorschaufeln 29 stehen bleibt, d.h. eine Rotationsströmung des Fluids F im Fluidraum 41 relativ zum stationären Filtergehäuse 2 wird zumindest erheblich reduziert, wenn nicht vollständig verhindert. Dies führt zu einer hohen Relativgeschwindigkeit zwischen der im Betriebszustand rotierenden Filtereinheit 3 und dem Fluid im Fluidraum 41. Diese hohe Relativgeschwindigkeit ist vorteilhaft, um ein Verstopfen des Filterelements 4 bzw. Ablagerungen auf oder in dem Filterelement 4 zu verhindern oder zumindest zu reduzieren.

Bei der in Fig. 12 dargestellten Variante ist der Einlass 23 für das Fluid F konzentrisch um den ersten Auslass 21 herum angeordnet, wobei der erste Auslass 21 wiederum im Zentrum der Stirnfläche 25 des Filtergehäuses 2 angeordnet ist, sodass er um die Solldrehachse herum angeordnet ist.

Ferner sind aussen auf der darstellungsgemäss (Fig. 12) oberen Stirnfläche 31 der Filtereinheit 3 die Pumpenflügel 8 zum Fördern des Fluids F vorgesehen, sodass die Filtereinheit 3 eine integrierte Pumpfunktion aufweist. Die Pumpenflügel 8 sind um die berührungslose Dichtung 7 herum angeordnet, wobei sich jeder Pumpenflügel 8 in radialer Richtung erstreckt.

Ist das Rotationsfiltersystem 1 beispielsweise mit einem Bioreaktor verbunden, so können die Pumpenflügel 8 zumindest dazu beitragen, das Fluid aus dem Bioreaktor durch den Einlass 23 anzusaugen und das Retentat durch den zweiten Auslass 22 zum Bioreaktor zu rezirkulieren. Je nach Ausgestaltung können die Pumpenflügel 8 auch die einzige Pumpvorrichtung zum Zirkulieren des Fluids F bzw. des Retentats R sein zwischen dem Bioreaktor und dem Rotationsfiltersystem 1 sein. Dazu sind die Pumpenflügel 8 mit einer grossen Erstreckung in radialer Richtung ausgestaltet, beispielsweise derart, dass sich die Pumpenflügel 8 bezüglich der radialen Richtung jeweils bis an die Umfangsfläche 32 der Filtereinheit 3 erstrecken. Zum einen dienen die Pumpenflügel 8 dazu, die Leckage durch die berührungslose Dichtung 7 zu reduzieren, und zum anderen erzeugen die Pumpenflügel 8 einen ausreichenden Druck und einen ausreichenden Fluss, um das Fluid durch das Rotationsfiltersystem 1 zu fördern.

Vorzugsweise ist bei dem erfindungsgemässen Rotationsfiltersystem 1 die Rotationsfiltervorrichtung 10, welche das Filtergehäuse 2 mit der darin angeordneten Filtereinheit 3 umfasst, als Einmalvorrichtung für den Einmalgebrauch ausgestaltet, und die Antriebsvorrichtung 100 ist als wiederverwendbare Vorrichtung für den Mehrfachgebrauch ausgestaltet. Hierzu ist es besonders vorteilhaft, dass - je nach Ausgestaltung - das Filtergehäuse 2 in sehr einfacher Weise in das Antriebsgehäuse 101 einsetzbar und von diesem trennbar ist, oder das Antriebsgehäuse 101 in sehr einfacher Weise in die zentrale Ausnehmung 20 des Filtergehäuses 2 einsetzbar bzw. aus dieser entfernbar ist. Somit können die als Einmalteil ausgestaltete Rotationsfiltervorrichtung 10 und die als wiederverwendbare Vorrichtung ausgestaltete Antriebsvorrichtung 100 in sehr einfacher Weise und vorzugsweise ohne die Verwendung eines Werkzeugs zusammengefügt und voneinander getrennt werden. Die Rotationsfiltervorrichtung 10 stellt dann als Einmalteil Verbrauchsmaterial dar, welches für genau eine Anwendung verwendet wird. Nach dieser Anwendung wird die Rotationsfiltervorrichtung 10 von der Antriebsvorrichtung 100 getrennt und entsorgt. Für die nächste Anwendung wird eine neue, das heisst unbenutzte Rotationsfiltervorrichtung 10 mit der Antriebsvorrichtung 100 zu dem Rotationsfiltersystem 1 zusammengesetzt.

Die Rotationsfiltervorrichtung 10 stellt somit eine separate Komponente des Rotationsfiltersystems 1 dar, die separat von der Antriebsvorrichtung 100 herstellbar und erwerbbar ist.

Durch die Erfindung wird ferner ein Separationssystem 200 für einen Bioreaktor 300 vorgeschlagen. Fig. 13 zeigt in einer schematischen Darstellung ein Ausführungsbeispiel des erfindungsgemässen Separationssystems 200, das gesamthaft mit dem Bezugszeichen 200 bezeichnet ist. Der Bioreaktor 300, für den das Separationssystem 200 geeignet ist, ist vorzugsweise als ein Perfusions-Bioreaktor 300 ausgestaltet. Ein Perfusions-Bioreaktor 300 wird beispielsweise zur kontinuierlichen Kultivierung von Zellen eingesetzt, wobei dann beispielsweise Stoffwechselprodukte der Zellen oder zellfreie Medien mittels Filtration separiert werden und die Zellen in den Bioreaktor 300 zurückgeführt werden. Dabei kann dem Bioreaktor 300 beispielsweise kontinuierlich eine Nährlösung für die Zellen zugeführt werden, wodurch die Masse oder das Volumen der ausgefilterten Komponenten ersetzt werden. Gerade bei solchen kontinuierlich ablaufenden Perfusionsprozessen ist es ein besonderer Vorteil des erfindungsgemässen Rotationsfiltersystems 1, dass das Filtergehäuse 2 als hermetisch dichtes Filtergehäuse 2 ausgestaltet werden kann und insbesondere keine dynamische Dichtung aufweist, die zwischen dem Innenraum des Filtergehäuses 2 und dem Aussenraum des Filtergehäuses 2 abdichten muss.

Das Separationssystem 200 für den Bioreaktor 300 zur Extraktion einer Substanz aus einem in dem Bioreaktor 300 vorrätigen Fluid, umfasst ein Rotationsfiltersystem 1, welches erfindungsgemäss ausgestaltet ist. Das Rotationsfiltersystem 1 umfasst den Einlass 23 für das Fluid F, den ersten Auslass 21 zum Abführen des Filtrats P, sowie den zweiten Auslass 22 zum Abführen des Retentats R. Beispielsweise ist das Filtrat P die zu extrahierende Substanz. Das Separationssystem 200 umfasst eine erste Strömungsverbindung 231, mit welcher der Einlass 23 mit dem Bioreaktor 300 verbindbar ist, sowie eine zweite Strömungsverbindung 232, mit welcher der zweite Auslass 22 mit dem Bioreaktor 300 verbindbar ist, wobei die Substanz als das Filtrat P durch den ersten Auslass 21 entnehmbar ist. Ferner ist eine Pumpvorrichtung 241 zum Zirkulieren des Fluids F und des Retentats R durch die erste Strömungsverbindung 231 und die zweite Strömungsverbindung 232 vorgesehen, wobei die Pumpvorrichtung 241 einen Einlass 245 und einen Auslass 246 aufweist.

Die Pumpvorrichtung 241 ist beispielsweise als Zentrifugalpumpe 241 ausgestaltet. Es sind aber auch solche Ausgestaltungen möglich, bei welchen die Pumpvorrichtung 241 in das Rotationsfiltersystem 1 integriert ist. Es ist möglich, die vorangehend beschriebenen Pumpenflügel 8, die an der Filtereinheit 3 des Rotationsfiltersystems 1 vorgesehen sein können, derart auszugestalten, dass sie die Pumpfunktion zur Zirkulation des Fluids F bzw. des Retentats R zwischen dem Bioreaktor 300 und dem Rotationsfiltersystem 1 vollständig übernehmen. Bei solchen Ausgestaltungen bilden dann also die Pumpenflügel 8 die Pumpvorrichtung 241. Ferner sind solche Ausgestaltungen möglich, bei denen die Pumpvorrichtung 241 eine separate, also von dem Rotationsfiltersystem 1 verschiedene Vorrichtung ist. Dabei ist es möglich, dass nur diese separate Pumpvorrichtung 241 die Zirkulation des Fluids F bzw. des Retentats R bewirkt, dass also beispielsweise an der Filtereinheit 3 keine Pumpenflügel 8 vorgesehen sind, oder dass sowohl die separate Pumpvorrichtung 241 vorgesehen ist als auch die Pumpenflügel 8, welche zu der Pumpfunktion beitragen. Es sind somit Ausgestaltungen möglich, bei welchen zur Zirkulation des Fluids F bzw. des Retentats R nur die separate Pumpvorrichtung 241 vorgesehen ist, sowie Ausgestaltungen, bei welchen keine separate Pumpvorrichtung 241 vorgesehen ist und die Pumpfunktion vollständig von dem Rotationsfiltersystem 1, beispielsweise den Pumpenflügeln 8 an der Filtereinheit 3, übernommen wird, sowie Ausgestaltungen, bei denen eine separate Pumpvorrichtung 241 vorgesehen ist, und das Rotationsfiltersystem 1 einen Beitrag zu der Pumpfunktion leistet, beispielsweise mittels der Pumpenflügel 8 an der Filtereinheit 3.

Bei solchen Ausgestaltungen, bei welchen eine separate, d.h. von dem Rotationsfiltersystem 1 verschiedene Pumpvorrichtung 241 vorgesehen ist, ist die Pumpvorrichtung 241 vorzugsweise als Zentrifugalpumpe 241 ausgestaltet, und besonders bevorzugt als eine Zentrifugalpumpe 241, die nach dem vorangehend erläuterten Prinzip des lagerlosen Motors ausgestaltet ist.

Dabei umfasst die Zentrifugalpumpe 241 einen Rotor zum Fördern des Fluids, sowie einen Stator, der mit dem Rotor einen elektromagnetischen Drehantrieb zum Rotieren des Rotors um eine axiale Richtung bildet, wobei der Rotor einen magnetisch wirksamen Kern umfasst, sowie eine Mehrzahl von Flügeln zum Fördern des Fluids, wobei der Stator als Lager- und Antriebsstator ausgestaltet ist, mit welchem der Rotor berührungslos magnetisch antreibbar und berührungslos magnetisch bezüglich des Stators lagerbar ist. Diese Ausgestaltung der Zentrifugalpumpe 241 mit einem magnetisch gelagerten Rotor, der gleichzeitig der Pumpenrotor der Zentrifugalpumpe ist und der Rotor des elektromagnetischen Drehantriebs zum Antreiben der Rotation, ermöglicht eine äusserst kompakte, platzsparende und leistungsfähige Ausgestaltung der Zentrifugalpumpe 241.

Gemäss einer besonders bevorzugten Ausgestaltung umfasst die Zentrifugalpumpe 241 eine Pumpeneinheit mit einem Pumpengehäuse, wobei das Pumpengehäuse einen Einlass und einen Auslass für das zu fördernde Fluid umfasst, wobei der Rotor im Pumpengehäuse angeordnet ist, und eine Mehrzahl von Flügeln zum Fördern des Fluids umfasst, und wobei die Pumpeneinheit derart ausgestaltet ist, dass die Pumpeneinheit in den Stator einsetzbar ist.

Durch die berührungslose magnetische Lagerung des Rotors bedarf es auch keiner mechanischen Lager, welche beispielsweise durch Abrieb zu Verunreinigungen des Fluids führen könnten. Auch ermöglicht die berührungslose magnetische Lagerung des Rotors eine äusserst präzise und einfache Einstellung des von der Zentrifugalpumpe 241 generierten Durchflusses, beispielsweise über die Drehzahl des Rotors.

Im Hinblick auf die magnetische Lagerung des Rotors der Zentrifugalpumpe 241 ist es besonders bevorzugt, dass der Rotor jeweils in einer zur axialen Richtung senkrechten radialen Ebene aktiv magnetisch gelagert ist, und in axialer Richtung sowie gegen Verkippungen passiv magnetisch stabilisiert ist.

Insbesondere kann der elektromagnetische Drehantrieb der Zentrifugalpumpe 241 auch als Tempelmotor ausgestaltet sein.

Die erste Strömungsverbindung 231 und die zweite Strömungsverbindung 232 werden vorzugsweise mit Leitungen realisiert, die als flexible Leitungen ausgestaltet sind, also als Leitungen, deren Wandung deformierbar ist. Jede Leitung ist beispielsweise als Schlauch, insbesondere als Kunststoffschlauch, ausgestaltet, der beispielsweise aus einem Silikonkautschuk, PVC (Polyvinylchlorid), PU (Polyurethan), PE (Polyethylen), HDPE (High Density Polyethylen), PP (Polypropylen), EVA (Ethyl Vinyl Acetat) oder Nylon besteht. Vorzugsweise ist jeder Schlauch, der zu der ersten 231 oder zu der zweiten Strömungsverbindung 232 gehört, für den Einmalgebrauch ausgestaltet. Bei der Ausgestaltung für den Einmalgebrauch werden diejenigen Komponenten, welche mit den zu behandelnden Substanzen in Kontakt kommen, hier also insbesondere die Schläuche, nur genau einmal verwendet und dann bei der nächsten Anwendung durch neue, das heisst ungebrauchte, Einmalteile ersetzt.

Es versteht sich, dass das Separationssystem 200 weitere Komponenten umfassen kann, beispielsweise Sensoren zum Erfassen von Druck oder Durchfluss oder Temperatur oder Viskosität. Üblicherweise ist auch eine Kontrolleinheit (in Fig. 13 nicht dargestellt) vorgesehen, mit welcher das Separationssystem 200 gesteuert oder geregelt wird.

Im Betriebszustand wird das Fluid F mittels der Pumpvorrichtung 241 aus dem Bioreaktor 300 durch die erste Strömungsverbindung 231 zum Einlass 23 des Rotationsfiltersystem 1 gefördert. Die zu extrahierende Substanz durchdringt das rotierende Filterelement 4 und wird anschliessend als Filtrat P durch den ersten Auslass 21 aus dem Filtratraum 42 abgeführt.

Das Retentat R wird mittels der Pumpvorrichtung 241 durch den zweiten Auslass 22 und die zweite Strömungsverbindung 232 zurück in den Bioreaktor 300 gefördert.

Fig. 14 zeigt in einer schematischen Darstellung eine Variante für das Ausführungsbeispiel des erfindungsgemässen Separationssystems 200.

In der zweiten Strömungsverbindung 232 ist eine zweite Zentrifugalpumpe 242 für das Fluid F bzw. für das Retentat R angeordnet, welche einen Einlass 243 und einen Auslass 244 für das Fluid bzw. das Retentat aufweist.

Dabei ist die zweite Zentrifugalpumpe 242 so angeordnet und wird so betrieben, dass sie entgegengesetzt zu der Pumpvorrichtung 241 arbeitet. Das heisst, dass der Auslass 246 der Pumpvorrichtung 241 über das Rotationsfiltersystem 1 mit dem Auslass 244 der zweiten Zentrifugalpumpe 242 verbunden ist. Sowohl der Auslass 246 der Pumpvorrichtung 241 als auch der Auslass 244 der zweiten Zentrifugalpumpe 242 sind jeweils mit dem Rotationsfiltersystem 1 verbunden.

Da die zweite Zentrifugalpumpe 242 entgegengesetzt zu der Pumpvorrichtung 241 arbeitet, kann die zweite Zentrifugalpumpe 242 einen Gegendruck an dem zweiten Auslass 22 des Rotationsfiltersystems 1 erzeugen, sodass der Druck an dem zweiten Auslass 22 grösser wird. Hierdurch erhöht sich der Druckabfall über das Filterelement 4, wodurch sich der Permeatstrom, also der Strom durch das Filterelement 4, vergrössern lässt. Durch die zweite Zentrifugalpumpe 242 lässt sich der Druck des Fluids an dem zweiten Auslass 22 mit hoher Genauigkeit, in einfacher Weise, reproduzierbar und über einen grossen Betriebsbereich zuverlässig einstellen.

Der Druck des Fluids an dem Einlass 23 des Rotationsfiltersystems 1 wird als ein erster Druck P1 bezeichnet. Der Druck des Retentats an dem zweiten Auslass 22 wird als ein zweiter Druck P2 bezeichnet Der Druck des Filtrats an dem ersten Auslass 21 wird als ein dritter Druck P3 bezeichnet. Der Transmembrandruck TMP wird dann wie allgemein üblich definiert als:

$$TMP = (P1+P2)/2 - P3$$

Der Transmembrandruck wird auch als transmembrane Druckdifferenz bezeichnet.

Natürlich ist es auch bei der in Fig. 14 dargestellten Variante möglich, dass die Pumpvorrichtung 241 vollständig in das Rotationsfiltersystem 1 integriert ist und dort durch die Pumpenflügel 8 realisiert wird, oder dass für die Pumpfunktion sowohl die separate Pumpvorrichtung 241 ausserhalb des Rotationsfiltersystems 1 als auch die Pumpenflügel 8 in dem Rotationsfiltersystem 1 vorgesehen sind.

Falls die Pumpenflügel 8 zum Erzeugen eines Drucks in der Rotationsfiltersystem 1 vorgesehen sind, so addiert sich der von den Pumpenflügeln 8 generierte Druck zu dem ersten Druck P1.

Alternativ oder ergänzend zu der zweiten Zentrifugalpumpe 242 kann in der zweiten Strömungsverbindung 232 auch ein ansteuerbares Kontrollventil 250 vorgesehen sein, mit welchem der Durchfluss durch die zweite Strömungsverbindung 232 einstellbar ist. Durch Einstellen des Durchflusses durch die zweite Strömungsverbindung 232 lässt sich ebenfalls der zweite Druck P2 einstellen. Wird beispielsweise bei konstant gehaltenem Betrieb der ersten Pumpvorrichtung 241 der Durchfluss durch die zweite Strömungsverbindung 232 reduziert, so erhöht sich dadurch der zweite Druck P2 am zweiten Auslass 22 des Rotationsfiltersystems 1.

Das optional vorgesehene Kontrollventil 250 kann entweder die zweite Zentrifugalpumpe 242 ersetzen, oder zusätzlich zu der zweiten Zentrifugalpumpe 242 vorgesehen sein. Falls, wie in Fig. 14 dargestellt, sowohl die zweite Zentrifugalpumpe 242 als auch das Kontrollventil 250 vorgesehen sind, so ist das Kontrollventil 250 zwischen dem Auslass 244 der zweiten Zentrifugalpumpe 242 und dem zweiten Auslass 22 des Rotationsfiltersystems 1 angeordnet.

Die erste Strömungsverbindung 231 umfasst einen Zuführschlauch 235, welcher eine erste Öffnung 301 des Bioreaktors 300 mit dem Einlass 245 der Pumpvorrichtung 241 verbindet, sowie einen Einspeiseschlauch 236, welcher den Auslass 246 der Pumpvorrichtung 241 mit dem Einlass 23 des Rotationsfiltersystems 1 verbindet. Falls keine separate Pumpvorrichtung 241 vorgesehen ist, können der Zuführschlauch 235 und der Einspeiseschlauch 236 als eine bauliche Einheit ausgestaltet sein, welche die erste Öffnung 301 mit dem Einlass 23 des Rotationsfiltersystems 1 verbindet.

Ferner ist ein Durchflusssensor 206 zur Ermittlung des Durchflusses des Fluids durch die erste Strömungsverbindung 231 vorgesehen. Der Durchflusssensor 206 ist beispielsweise in oder an dem Einspeiseschlauch 236 der ersten Strömungsverbindung 231 vorgesehen. Natürlich ist es auch möglich, den Durchflusssensor 206 in oder an dem Zuführschlauch 235 vorzusehen, also zwischen dem Bioreaktor 300 und der Pumpvorrichtung 241. Insbesondere kann der Durchflusssensor 206 als sogenannter Clamp-on Sensor ausgestaltet sein, das heisst als ein Durchflusssensor 206, der auf den Einspeiseschlauch 236 bzw. auf den Zuführschlauch 235 aufgeklemmt wird, sodass der Einspeiseschlauch 236 bzw. der Zuführschlauch 235 in dem Messbereich des Durchflusssensors 206 eingeklemmt ist.

Die zweite Strömungsverbindung 232 umfasst einen Abführschlauch 238, welcher den zweiten Auslass 22 des Rotationsfiltersystems 1 mit dem Kontrollventil 250, oder, falls dieses nicht vorgesehen ist, mit dem Auslass 244 der zweiten Zentrifugalpumpe 242 verbindet.

Die zweite Strömungsverbindung 232 umfasst ferner einen Rückführschlauch 239, welcher den Einlass 243 der zweiten Zentrifugalpumpe 242 mit einer zweiten Öffnung 302 des Bioreaktors 300 verbindet. Falls die zweite Zentrifugalpumpe 242 nicht vorgesehen ist, verbindet der Rückführschlauch 239 die zweite Öffnung 302 des Bioreaktors 300 mit dem Kontrollventil 250.

Somit sind sowohl der Auslass 246 der Pumpvorrichtung 241 als auch der Auslass 242 der zweiten Zentrifugalpumpe 242 oder das Kontrollventil 250 jeweils mit dem Rotationsfiltersystem 1 verbunden, nämlich mit dem Einlass 23 bzw. mit dem zweiten Auslass 22 des Rotationsfiltersystems 1. Daher kann mittels der zweiten Zentrifugalpumpe 242 und/oder mittels des Kontrollventils 250 an dem zweiten Auslass 22 einen Gegendruck erzeugt werden, sodass an dem zweiten Auslass 22 der zweite Druck P2 eingestellt werden kann.

Im Betriebszustand dienen die Pumpvorrichtung 241 und/oder die Pumpenschaufeln 8 des Rotationsfiltersystems 1 dazu, das Fluid durch das Rotationsfiltersystem 1 und über das Filterelement 4 zu bewegen. Die Pumpvorrichtung 241 und/oder die Pumpenschaufeln 8 zirkulieren das Fluid aus dem Bioreaktor 300 durch die erste Strömungsverbindung 231, durch das Rotationsfiltersystem 1 und als Retentat durch die zweite Strömungsverbindung 232 zurück in den Bioreaktor 300.

Im Betriebszustand dient die zweite Zentrifugalpumpe 242 und/oder das Kontrollventil 250 dazu, an dem zweiten Auslass 22 des Rotationsfiltersystems 1 einen Gegendruck zu generieren, das heisst, die zweite Zentrifugalpumpe 242 und/oder das Kontrollventil 250 werden derart betrieben, dass sie den zweiten Druck P2, welcher an dem zweiten Auslass 22 herrscht, erhöhen.

Vorzugsweise umfasst das Separationssystem 200 ferner eine Mehrzahl von Drucksensoren 271, 272, 273, wobei die Drucksensoren 271, 272 und 273 vorzugsweise so angeordnet und ausgestaltet sind, dass mit Ihnen der Transmembrandruck über das Filterelement 4 ermittelbar ist.

Bei der in Fig. 14 dargestellten Variante sind insgesamt drei Drucksensoren 271, 272, 273 vorgesehen, nämlich ein erster Drucksensor 271, mit welchem der erste Druck P1 des Fluids an dem Einlass 23 des Rotationsfiltersystems 1 ermittelbar ist, ein zweiter Drucksensor 272, mit welchem der zweite Druck P2 des Retentats an dem zweiten Auslass 22 des Rotationsfiltersystems 1 ermittelbar ist, und ein dritter Drucksensor 273, mit welchem der dritte Druck P3 an dem ersten Auslass 21 des Rotationsfiltersystems 1 ermittelbar ist.

Bei der in Fig. 14 dargestellten Variante ist der erste Drucksensor 271 zwischen dem Durchflusssensor 206 und dem Einlass 23 des Rotationsfiltersystems 1 vorgesehen, nämlich in oder an dem Einspeiseschlauch 236. Der zweite Drucksensor 272 ist zwischen dem zweiten Auslass 22 des Rotationsfiltersystems 1 und dem Kontrollventil 250 bzw. der zweiten Zentrifugalpumpe 242 vorgesehen, nämlich in oder an dem Abführschlauch 238. Der dritte Drucksensor 273 ist an oder stromabwärts des ersten Auslasses 21 in oder an einer Filtratleitung 210 angeordnet, durch welche das Filtrat P vom ersten Auslass 21 abgeführt wird.

In oder an der Filtratleitung 210 ist optional ein zweiter Durchflusssensor 207 vorgesehen, mittels welchem der Durchfluss des Filtrats P durch die Filtratleitung 210 ermittelbar ist.

Ferner ist optional in der Filtratleitung 210 eine dritte Pumpvorrichtung 211 vorgesehen, um das Filtrat P durch die Filtratleitung 210 zu fördern. Der zweite Durchflusssensor 207 und der dritte Drucksensor 273 sind vorzugsweise stromaufwärts der dritten Pumpvorrichtung 211 angeordnet. Die dritte Pumpvorrichtung 211 ist beispielsweise als peristaltische Pumpe ausgestaltet.

Ferner ist eine Kontrolleinheit 205 vorgesehen, mit welcher das Separationssystem 200 betrieben und angesteuert bzw. geregelt wird. Dazu ist die Kontrolleinheit 205 mit den verschiedenen Komponenten des Separationssystems signalverbunden. Die Signalverbindungen sind in Fig. 14 durch strichliert dargestellte Pfeile angedeutet und können jeweils als physische Verbindungen, also beispielsweise als Signalkabel oder Signalleitungen, ausgestaltet sein oder als drahtlose (wireless) Signalverbindungen.

Die Signalverbindungen S1, S2 und S3 verbinden die Kontrolleinheit 205 mit der ersten Pumpvorrichtung 241, der zweiten Zentrifugalpumpe 242 und der dritten Pumpvorrichtung 211. Mit diesen Signalverbindungen S1, S2, S3 werden die Pumpvorrichtungen 241, 242, 211 angesteuert, beispielsweise wird die Drehzahl oder der zu generierende Fluss gesteuert oder geregelt. Die Signalverbindung S4 verbindet die Kontrolleinheit 205 mit dem Rotationsfiltersystem 1 und dient beispielsweise dazu, die Drehzahl der Filtereinheit 3 einzustellen oder zu regeln. Die Signalverbindung S5 dient der Ansteuerung des Kontrollventils 250. Über die Signalverbindung S5 kann der Durchfluss durch das Kontrollventil 250 eingestellt werden. Die Signalverbindungen S6, S7, S8 dienen dem Datenaustausch mit den Drucksensoren 271, 272, 273. Über die Signalverbindungen S6, S7, S8 können die Drucksensoren 271, 272, 273 ihre jeweiligen Messwerte an die Kontrolleinheit 205 übermitteln. Die Signalverbindungen S9, S10 dienen dem Datenaustausch mit den Durchflusssensoren 206, 207. Über die Signalverbindungen S9 und S10 können die Durchflusssensoren 206, 207 ihre jeweiligen Messwerte an die Kontrolleinheit 205 übermitteln.

Vorzugsweise, aber nicht notwendigerweise ist die zweite Zentrifugalpumpe 242 zumindest im Wesentlichen identisch ausgestaltet wie die Pumpvorrichtung 241, die -wie bereits gesagt vorzugsweise auch als Zentrifugalpumpe 242 ausgestaltet ist. Insbesondere ist es daher bevorzugt, dass sowohl die Pumpvorrichtung 241 als auch die zweite Zentrifugalpumpe 242 jeweils als eine Zentrifugalpumpe 241, 242 ausgestaltet sind, die nach dem bereits erläuterten Prinzip des lagerlosen Motors ausgestaltet sind.

In sinngemäss gleicher Weise wie dies bereits für das erfindungsgemässe Rotationsfiltersystem 1 erläutert wurde, kann auch das Separationssystem 200 derart ausgestaltet sein, dass es ein wiederverwendbares System umfasst, das für den Mehrfachgebrauch ausgestaltet ist, sowie eine Einmalsystem, das für den Einmalgebrauch ausgestaltet ist. Das wiederverwendbare System umfasst dabei insbesondere diejenigen Komponenten, welche nicht mit dem Fluid bzw. dem Retentat bzw. dem Filtrat in Berührung kommen, also insbesondere die Antriebsvorrichtung 100 des Rotationsfiltersystems 1, die Statoren der Zentrifugalpumpen 241, 242 und beispielsweise zumindest Teile der Drucksensoren 271, 272, 273. Die Drucksensoren 271, 272, 273 können dabei so ausgestaltet sein, dass sie jeweils Einmalteile umfassen und wiederverwendbare Teile.

Das Einmalsystem, welches die Komponenten umfasst, die für den Einmalgebrauch ausgestaltet sind, umfasst zumindest die folgenden Komponenten: die Rotationsfiltervorrichtung 10, die Pumpeneinheiten für jede Zentrifugalpumpe 241, 242, eine Mehrzahl von Schläuchen 235, 236, 238, 239, welche zum Realisieren der ersten Strömungsverbindung 231 und der zweiten Strömungsverbindung 232ausgestaltet ist, und optional mindestens einen Schlauch für die Filtratleitung 210.

Ein weiterer wesentliche Aspekt ist es, dass alle Teile des Rotationsfiltersystems 1 und des Separationssystems 200, welche das Fluid F oder das Retentat R oder das Filtrat P berühren, insbesondere die Rotationfiltervorrichtung 10, die Strömungsverbindungen 231, 232, gegebenenfalls die Drucksensoren 271, 272, 273 und die Pumpeneinheiten der Zentrifugalpumpen 241 und 242 bzw. ihre Komponenten für gewisse Anwendungsbereiche sterilisierbar sein sollen. Dabei ist es besonders vorteilhaft, wenn alle genannten Komponenten gammasterilisierbar sind. Bei dieser Art der Sterilisierung wird die zu sterilisierende Komponente mit Gamma-Strahlung beaufschlagt. Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen ist es eine gängige Praxis, dass die Teile nach ihrer Herstellung in die für den Versand vorgesehene Verpackung gebracht werden und dann noch eine Zeit lagern, bevor sie an den Kunden ausgeliefert werden. In solchen Fällen erfolgt die Sterilisierung erst kurz vor der Auslieferung an den Kunden durch die Verpackung hindurch, was bei einer Dampfsterilisierung oder anderen Verfahren nicht möglich ist.

Bezüglich der Einmalteile ist es in der Regel nicht notwendig, dass sie mehr als einmal sterilisierbar sein müssen. Dies ist insbesondere bei der Gamma-Sterilisierung ein grosser Vorteil, weil die Beaufschlagung mit Gamma-Strahlung bei Kunststoffen zu Degradationen führen kann, sodass eine mehrfache Gamma-Sterilisierung den Kunststoff unbrauchbar machen kann.

Da in der Regel bei Einmalteilen auf eine Sterilisierung unter hohen Temperaturen und /oder unter hohem (Dampf-) Druck verzichtet werden kann, können kostengünstigere Kunststoffe eingesetzt werden, beispielsweise solche, die keine hohen Temperaturen aushalten, oder die nicht mehrfach hohen Temperatur- und Druckwerten ausgesetzt werden können.

Unter Berücksichtigung all dieser Aspekte ist es daher bevorzugt, für die Herstellung der Einmalteile solche Kunststoffe zu verwenden, die zumindest einmal gamma-sterilisierbar sind.

Die Materialien sollten dabei gammastabil für eine Dosis von mindestens 40 kGy sein, um eine einmalige Gamma-Sterilisierung zu ermöglichen. Bei der Gamma-Sterilisierung sollten zudem keine giftigen Stoffe entstehen. Zudem ist es bevorzugt, wenn alle Materialien, die mit den zu mischenden bzw. den durchmischten Substanzen in Berührung kommen, USP Class VI Standards erfüllen.

Für die Herstellung der aus Kunststoff bestehenden Teile, z.B. das Filtergehäuse 2 des Rotationsfiltersystems 1, sind beispielsweise folgende Kunststoffe bevorzugt: PolyEthylene (PE), PolyPropylene (PP), Low Density PolyEthylene (LDPE), Ultra Low Density PolyEthylene (ULDPE), Ethylene Vinyl Acetate (EVA), PolyEthylene Terephthalate (PET), PolyVinylChlorid (PVC), PolyVinyliDene Fluoride (PVDF), Acrylonitrile Butadiene Styrene (ABS), PolyAcryl, PolyCarbonate (PC).

Weniger geeignete oder sogar ungeeignete Materialien für die Herstellung der Kunststoffteile der Einmalkomponenten sind beispielsweise die unter dem Markennamen Teflon bekannten Materialien Polytetrafluoroethylene (PTFE) und und Perfluoralkoxy-Polymere (PFA). Bei diesen Materialien besteht nämlich bei der Gamma-Sterilisierung die Gefahr, das gefährliche Gase austreten, wie beispielsweise Fluor, das dann giftige oder schädliche Verbindungen wie Flusssäure (HF) bilden kann.

**Patentansprüche**

1. Rotationsfiltersystem zum Ausfiltern eines Filtrats aus einem Fluid, mit einer Rotationsfiltervorrichtung (10) und einer Antriebsvorrichtung (100), wobei die Rotationsfiltervorrichtung (10) ein stationäres Filtergehäuse (2) und eine um eine axiale Richtung (A) rotierbare Filtereinheit (3) umfasst, wobei das Filtergehäuse (2) einen Einlass (23) für das Fluid (F), einen ersten Auslass (21) zum Abführen des Filtrats (P), sowie einen zweiten Auslass (22) zum Abführen eines Retentats (R) aufweist, wobei die Filtereinheit (3) in dem Filtergehäuse (2) angeordnet und vollständig von dem Filtergehäuse (2) umschlossen ist, wobei die Filtereinheit (3) ein Filterelement (4) aufweist, welches einen Fluidraum (41) von einem Filtratraum (42) abgrenzt, wobei das Filtrat (P) aus dem Filtratraum (42) durch den ersten Auslass (21) abführbar ist, wobei die Filtereinheit (3) ferner einen magnetisch wirksamen Kern (6, 6') umfasst, welcher scheiben- oder ringförmig ausgestaltet ist, und wobei die Antriebsvorrichtung (100) ein Antriebsgehäuse (101) aufweist, in welchem ein Stator (102, 102') zum Antreiben der Rotation der Filtereinheit (3) vorgesehen ist, **dadurch gekennzeichnet, dass** der Stator (102, 102') als Lager- und Antriebsstator ausgestaltet ist, der mit dem magnetisch wirksamen Kern (6, 6') der Filtereinheit (3) als elektromagnetischer Drehantrieb zusammenwirkt, sodass die Filtereinheit (3) berührungslos magnetisch antreibbar und bezüglich des Stators (102, 102') magnetisch lagerbar ist.

2. Rotationsfiltersystem nach Anspruch 1, wobei das Filtergehäuse (2) zum hermetischen Umschliessen der Filtereinheit (3) ausgestaltet ist.

3. Rotationsfiltersystem nach einem der vorangehenden Ansprüche, wobei zwischen der rotierbaren Filtereinheit (3) und dem ersten Auslass (21) des Filtergehäuses (2) eine berührungslose Dichtung (7) vorgesehen ist, und wobei die berührungslose Dichtung (7) im Innenraum des Filtergehäuses (2) angeordnet ist.

4. Rotationsfiltersystem nach Anspruch 3, wobei auf der Filtereinheit (3) und benachbart zu der berührungslosen Dichtung (7) Pumpenflügel (8) zum Fördern des Fluids vorgesehen sind, wobei die Pumpenflügel (8) zur Rotation um die axiale Richtung (A) ausgestaltet und drehfest mit der Filtereinheit (3) verbunden sind.

5. Rotationsfiltersystem nach einem der vorangehenden Ansprüche, bei welchem an einer Aussenseite (31, 32) der Filtereinheit (3) Schaufeln (9) zum Erzeugen eines Transmembrandrucks über das Filterelement (4) vorgesehen sind, wobei die Schaufeln (9) zur Rotation um die axiale Richtung (A) ausgestaltet und drehfest mit der Filtereinheit (3) verbunden sind.

6. Rotationsfiltersystem nach einem der vorangehenden Ansprüche, wobei das Filtergehäuse (2) eine Stirnfläche (25) aufweist, an welcher der Einlass (23), der erste Auslass (21) und der zweite Auslass (22) angeordnet sind.

7. Rotationsfiltersystem nach einem der vorangehenden Ansprüche, wobei die Filtereinheit (3) zwei magnetisch wirksame Kerne (6, 6') umfasst, von denen jeder scheiben- oder ringförmig ausgestaltet ist, und die bezüglich der axialen Richtung (A) beabstandet zueinander angeordnet sind, wobei im Antriebsgehäuse (101) der Antriebsvorrichtung (100) zwei Statoren (102, 102') zum Antreiben der Rotation der Filtereinheit (3) vorgesehen sind, wobei jeder Stator (102, 102') als Lager- und Antriebsstator ausgestaltet ist, der mit jeweils einem der magnetisch wirksamen Kerne (6, 6') der Filtereinheit (3) als elektromagnetischer Drehantrieb zusammenwirkt, sodass die Filtereinheit (3) berührungslos magnetisch antreibbar und bezüglich der Statoren (102, 102') magnetisch lagerbar ist.

8. Rotationsfiltersystem nach einem der vorangehenden Ansprüche, wobei die Rotationsfiltervorrichtung (10) und die Antriebsvorrichtung (100) so ausgestaltet sind, dass das stationäre Filtergehäuse (2) in das Antriebsgehäuse (101) einsetzbar ist, und jeder elektromagnetische Drehantrieb als Innenläufer ausgestaltet ist.

9. Rotationsfiltersystem nach einem der Ansprüche 1-7, wobei die Rotationsfiltervorrichtung (10) und die Antriebsvorrichtung (100) so ausgestaltet sind, dass das Antriebsgehäuse (101) in eine zentrale Ausnehmung (20) des stationären Filtergehäuses (2) einsetzbar ist, und jeder elektromagnetische Drehantrieb als Aussenläufer ausgestaltet ist.

10. Rotationsfiltersystem nach einem der vorangehenden Ansprüche, bei welchem die Rotationsfiltervorrichtung (10) als Einmalvorrichtung für den Einmalgebrauch ausgestaltet ist, und bei welchem die Antriebsvorrichtung (100) als wiederverwendbare Vorrichtung für den Mehrfachgebrauch ausgestaltet ist, wobei das Filtergehäuse (2) in das Antriebsgehäuse (101) einsetzbar ist, oder das Filtergehäuse (2) eine zentrale Ausnehmung (20) zur Aufnahme des Antriebsgehäuses (101) aufweist.

11. Rotationsfiltervorrichtung für ein Rotationsfiltersystem, welches gemäss einem der vorangehenden Ansprüche ausgestaltet ist, wobei das Filtergehäuse (2) in das Antriebsgehäuse (101) einsetzbar ist, oder das Filtergehäuse (2) eine zentrale Ausnehmung (20) zur Aufnahme des Antriebsgehäuses aufweist.

12. Rotationsfiltervorrichtung nach Anspruch 11, ausgestaltet als Einmalvorrichtung für den Einmalgebrauch.

13. Separationssystem für einen Bioreaktor zur Extraktion einer Substanz aus einem in dem Bioreaktor vorrätigen Fluid, wobei das Separationssystem (200) eine Rotationsfiltersystem (1) umfasst, welches einen Einlass (23) für das Fluid (F), einen ersten Auslass (21) zum Abführen eines Filtrats (P), sowie einen zweiten Auslass (22) zum Abführen eines Retentats (R) aufweist, wobei eine erste Strömungsverbindung (231) vorgesehen ist, mit welcher der Einlass (23) mit dem Bioreaktor (300) verbindbar ist, wobei eine zweite Strömungsverbindung (232) vorgesehen ist, mit welcher der zweite Auslass (22) mit dem Bioreaktor (300) verbindbar ist, wobei die Substanz als das Filtrat (P) durch den ersten Auslass (21) entnehmbar ist, und wobei eine Pumpvorrichtung (241, 8) zum Zirkulieren des Fluids und des Retentats durch die erste Strömungsverbindung (231) und die zweite Strömungsverbindung (232) vorgesehen ist, **dadurch gekennzeichnet, dass** das Rotationsfiltersystem (1) nach einem der Ansprüche 1-10 ausgestaltet ist.

14. Separationssystem nach Anspruch 13, bei welchem die Pumpvorrichtung (8) in das Rotationsfiltersystem (1) integriert ist.

15. Separationssystem nach Anspruch 14, bei welchem die Pumpvorrichtung (8) an der rotierbaren Filtereinheit (3) vorgesehen ist.

Fig.1

Fig.2

Fig.3

Fig.4

Fig.5

Fig.6

Fig.7

Fig.8

Fig.9

Fig.10

Fig.11

Fig.12

Fig.13

Fig.14

Europäisches
Patentamt

European
Patent Office

Office européen
des brevets

# EUROPÄISCHER RECHERCHENBERICHT

Nummer der Anmeldung

EP 23 17 6800

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 86/02858 A (BAXTER INT INC) 22. Mai 1986 (1986-05-22) | 1-4,6, 8-15 | INV. B01D63/16 |
| Y | * Seite 7, Zeilen 5-15; Abbildung 1 * * Seite 1, Zeilen 3-6 * ───── | 4,5 | B01D65/08 B01D33/00 B01D46/00 |
| X | US 4 816 151 A (SCHOENDORFER DONALD W [US] ET AL) 28. März 1989 (1989-03-28) | 1-4,6, 8-15 | B01D46/26 |
| Y | * Spalte 1, Zeilen 11-14 * * Spalte 1, Zeilen 39-56 * ───── | 4 | |
| X | US 2006/054549 A1 (SCHOENDORFER DONALD W [US]) 16. März 2006 (2006-03-16) * Absatz [0012]; Ansprüche 1,9; Abbildungen 1,4 * ───── | 1-4,6, 8-15 | |
| A | US 2020/316501 A1 (JONES GARETH EVAN LYN [GB]) 8. Oktober 2020 (2020-10-08) * Absatz [0069] * ───── | 1-4,6, 8-15 | |
| A | US 2019/118127 A1 (WILLIMEK NORMANN [DE] ET AL) 25. April 2019 (2019-04-25) * Absatz [0054] * ───── | 1-4,6, 8-15 | RECHERCHIERTE SACHGEBIETE (IPC) |
| A | WO 91/01796 A1 (YT LI ENGINEERING INC [US]) 21. Februar 1991 (1991-02-21) * Seiten 1,2 * ───── | 1-4,6, 8-15 | B01D |
| A | US 4 230 564 A (KEEFER BOWIE G) 28. Oktober 1980 (1980-10-28) * Spalte 12, Zeilen 34-54; Anspruch 22; Abbildung 4 * ───── | 1-4,6, 8-15 | |
| A | US 5 603 831 A (HICKOK ROY S [US]) 18. Februar 1997 (1997-02-18) * Anspruch 18; Abbildung 11 * ───── | 1-4,6, 8-15 | |

-/--

Der vorliegende Recherchenbericht wurde für alle Patentansprüche erstellt

| Recherchenort | Abschlußdatum der Recherche | Prüfer |
|---|---|---|
| München | 3. August 2023 | Hennebrüder, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 ..............................................................................
& : Mitglied der gleichen Patentfamilie, übereinstimmendes Dokument

EPO FORM 1503 03.82 (P04C03)

**EP 4 292 694 A1**

Europäisches Patentamt
European Patent Office
Office européen des brevets

# EUROPÄISCHER RECHERCHENBERICHT

Nummer der Anmeldung

EP 23 17 6800

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2019/263678 A1 (NAZZER CRAIG [NZ]) 29. August 2019 (2019-08-29) * Absatz [0101]; Abbildung 3 * ----- | 1-4,6, 8-15 | |
| Y | WO 03/051496 A1 (SHINKO PANTEC KABUSHIKI KAISHA [JP]) 26. Juni 2003 (2003-06-26) * Absätze [0204] – [0206], [0128]; Abbildungen 3, 2b * ----- | 5 | |
| Y | HARSCOAT C ET AL: "Influence of fermentation conditions and microfiltration processes on membrane fouling during recovery of glucuronane polysaccharides from fermentation broths", BIOTECHNOLOGY AND BIOENGINEERING, JOHN WILEY, HOBOKEN, USA, Bd. 65, Nr. 5, 26. März 2000 (2000-03-26), Seiten 500-511, XP071051946, ISSN: 0006-3592, DOI: 10.1002/(SICI)1097-0290(19991205)65:5<500::AID-BIT2>3.0.CO;2-N * 1. vollständiger Satz; Seite 508, rechte Spalte * ----- | 5 | |
| Y | US 2016/175779 A1 (DAVIE RICHARD [US] ET AL) 23. Juni 2016 (2016-06-23) * Absatz [0068]; Abbildungen 15,16 * ----- | 5 | RECHERCHIERTE SACHGEBIETE (IPC) |
| Y | GB 1 308 165 A (EC CORP) 21. Februar 1973 (1973-02-21) * Seite 2, rechte Spalte, Zeilen 111-125; Abbildungen 1,2 * ----- | 4 | |
| X | CH 673 407 A5 (SULZER AG) 15. März 1990 (1990-03-15) * Seite 3, Zeile 36ff; Anspruch 1; Abbildungen 1,2 * ----- | 1,7 | |

Der vorliegende Recherchenbericht wurde für alle Patentansprüche erstellt

| Recherchenort | Abschlußdatum der Recherche | Prüfer |
|---|---|---|
| München | 3. August 2023 | Hennebrüder, K |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

Seite 2 von 2

Europäisches
Patentamt

European
Patent Office

Office européen
des brevets

**Nummer der Anmeldung**

**EP 23 17 6800**

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

**Siehe Ergänzungsblatt B**

☐ Alle weiteren Recherchengebühren wurden innerhalb der gesetzten Frist entrichtet. Der vorliegende europäische Recherchenbericht wurde für alle Patentansprüche erstellt.

☒ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

**Nummer der Anmeldung**

**EP 23 17 6800**

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

```
    1. Ansprüche: 1-15

        Rotationsfiltersystem mit einem elektromagnetischen
        Drehantrieb
        mit Pumpenflügel (8) zum Fördern des Fluids


    1.1. Anspruch: 5

        Rotationsfiltersystem mit einem elektromagnetischen
        Drehantrieb mit Schaufel (9) zum Erzeugen des TMP
        (Transmembrandruck, trans membrane pressure TMP)


    1.2. Anspruch: 7

        Rotationsfiltersystem mit einem elektromagnetischen
        Drehantrieb mit Schaufel (9) zum Erzeugen des TMP
        (Transmembrandruck, trans membrane pressure TMP)
        mit zwei Statoren
                        ---


Bitte zu beachten dass für alle unter Punkt 1 aufgeführten Erfindungen,
obwohl diese nicht unbedingt durch ein gemeinsames erfinderisches
Konzept verbunden sind, ohne Mehraufwand der eine zusätzliche
Recherchengebühr gerechtfertigt hätte, eine vollständige Recherche
durchgeführt werden konnte.
```

**ANHANG ZUM EUROPÄISCHEN RECHERCHENBERICHT**
**ÜBER DIE EUROPÄISCHE PATENTANMELDUNG NR.**

`EP 23 17 6800`

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

`03-08-2023`

| Im Recherchenbericht angeführtes Patentdokument | Datum der Veröffentlichung | Mitglied(er) der Patentfamilie | Datum der Veröffentlichung |
|---|---|---|---|
| WO 8602858 A | 22-05-1986 | ------------------------------------ | |
| US 4816151 A | 28-03-1989 | KEINE | |
| US 2006054549 A1 | 16-03-2006 | AT 401947 T | 15-08-2008 |
| | | AU 2003240937 A1 | 02-02-2004 |
| | | CA 2485227 A1 | 22-01-2004 |
| | | EP 1509306 A1 | 02-03-2005 |
| | | JP 4377813 B2 | 02-12-2009 |
| | | JP 2005528215 A | 22-09-2005 |
| | | US 2006054549 A1 | 16-03-2006 |
| | | WO 2004007048 A1 | 22-01-2004 |
| US 2020316501 A1 | 08-10-2020 | AU 2018390481 A1 | 09-07-2020 |
| | | CA 3085969 A1 | 27-06-2019 |
| | | CN 111741801 A | 02-10-2020 |
| | | EP 3727639 A1 | 28-10-2020 |
| | | EP 4066916 A2 | 05-10-2022 |
| | | JP 2021506576 A | 22-02-2021 |
| | | KR 20200096279 A | 11-08-2020 |
| | | TW 201932178 A | 16-08-2019 |
| | | US 2020316501 A1 | 08-10-2020 |
| | | WO 2019122862 A1 | 27-06-2019 |
| US 2019118127 A1 | 25-04-2019 | DE 102017109104 A1 | 31-10-2018 |
| | | RU 2018115538 A | 25-10-2019 |
| | | US 2019118127 A1 | 25-04-2019 |
| WO 9101796 A1 | 21-02-1991 | KEINE | |
| US 4230564 A | 28-10-1980 | KEINE | |
| US 5603831 A | 18-02-1997 | CA 2190165 A1 | 30-11-1995 |
| | | DE 69519570 T2 | 07-06-2001 |
| | | EP 0762921 A1 | 19-03-1997 |
| | | JP 2755829 B2 | 25-05-1998 |
| | | JP H09507644 A | 05-08-1997 |
| | | US 5603831 A | 18-02-1997 |
| | | WO 9532046 A1 | 30-11-1995 |
| US 2019263678 A1 | 29-08-2019 | AU 2015307326 A1 | 23-03-2017 |
| | | CA 2959308 A1 | 03-03-2016 |
| | | EP 3194343 A2 | 26-07-2017 |
| | | US 2018222769 A1 | 09-08-2018 |
| | | US 2019263678 A1 | 29-08-2019 |
| | | WO 2016032344 A2 | 03-03-2016 |

EPO FORM P0461

Für nähere Einzelheiten zu diesem Anhang : siehe Amtsblatt des Europäischen Patentamts, Nr.12/82

`Seite 1 von 2`

**ANHANG ZUM EUROPÄISCHEN RECHERCHENBERICHT
ÜBER DIE EUROPÄISCHE PATENTANMELDUNG NR.**

EP 23 17 6800

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-08-2023

| Im Recherchenbericht angeführtes Patentdokument | Datum der Veröffentlichung | Mitglied(er) der Patentfamilie | Datum der Veröffentlichung |
|---|---|---|---|
| WO 03051496 A1 | 26-06-2003 | TW 550113 B<br>WO 03051496 A1 | 01-09-2003<br>26-06-2003 |
| US 2016175779 A1 | 23-06-2016 | CA 2957443 A1<br>CN 105934270 A<br>CN 109331661 A<br>CN 109331662 A<br>EP 3237098 A1<br>EP 3760300 A1<br>TW 201625344 A<br>TW 202033265 A<br>US 2016175779 A1<br>US 2018194647 A1<br>US 2019225511 A1<br>WO 2016106130 A1 | 30-06-2016<br>07-09-2016<br>15-02-2019<br>15-02-2019<br>01-11-2017<br>06-01-2021<br>16-07-2016<br>16-09-2020<br>23-06-2016<br>12-07-2018<br>25-07-2019<br>30-06-2016 |
| GB 1308165 A | 21-02-1973 | KEINE | |
| CH 673407 A5 | 15-03-1990 | CH 673407 A5<br>EP 0296336 A1<br>JP S6422312 A<br>US 4900440 A | 15-03-1990<br>28-12-1988<br>25-01-1989<br>13-02-1990 |

EPO FORM P0461

Für nähere Einzelheiten zu diesem Anhang : siehe Amtsblatt des Europäischen Patentamts, Nr.12/82

Seite 2 von 2